# EUROPEAN PATENT SPECIFICATION

(11) **EP 2 316 327 B1**
(45) Date of publication and mention of the grant of the patent: **15.05.2013**
(21) Application number: 10015493.9
(22) Date of filing: 14.10.2009
(51) Int. Cl.: A61B 1/00, A61B 1/04

(54) **Image display device, image display method, and image display program**
Bildanzeigevorrichtung, Bildanzeigeverfahren und Bildanzeigeprogramm
Dispositif d'affichage d'images, procédé d'affichage d'images et programme d'affichage d'images

(30) Priority: 14.10.2008 JP 2008265699; 21.10.2008 JP 2008271268
(43) Date of publication of application: 04.05.2011
(62) Divisional of application: 09012976.8
(73) Proprietor: Olympus Medical Systems Corp., Tokyo 151-0072 (JP)
(72) Inventor: Nishiyama, Takeshi, Tokyo 151-0072 (JP); Oguri, Atsushi, Tokyo 151-0072 (JP); Zui, Eri, Tokyo 151-0072 (JP)
(74) Representative: Gunzelmann, Rainer

(56) References cited:
- EP-A1- 1 787 574
- EP-A1- 1 922 977
- EP-A2- 1 857 042
- JP-A- 2005 131 031
- JP-A- 2006 301 535
- US-A1- 2003 228 133

## Description

### TECHNICAL FIELD

The present invention relates to an image display device for displaying a series of images of the inside of organs of a subject such as a patient captured in time-series. The present invention particularly relates to an image display device capable of displaying a list of a plurality of images in the series of images.

### BACKGROUND ART

In the field of endoscopes, a capsule endoscope, which is a swallow-type endoscope and has a capturing function and a wireless communication function in its capsule-shaped casing, has been developed. The capsule endoscope is inserted into an organ of a subject such as a patient through oral ingestion, etc. The capsule endoscope inside the subject then captures images (hereinafter sometimes referred to as in-vivo images) of the inside of the organ of the subject and wirelessly transmits the obtained in-vivo images to the outside while moving through the organs by peristaltic motion, etc. During the period from when inserted into the subject until excreted to the outside of the subject, such capsule endoscope sequentially captures the in-vivo images in time-series, and wirelessly transmits the obtained in-vivo images to the outside of the subject in time-series order.

Each in-vivo image wirelessly transmitted in time-series order by such capsule endoscope is sequentially received by a receiving device at the exterior of the subject. The receiving device saves the group of in-vivo images received in time-series order from such capsule endoscope in a recording medium inserted in advance. Thereafter, the recording medium storing such in-vivo image group is detached from the receiving device and inserted to the image display device. The image display device takes the in-vivo image group in the inserted recording medium, and sequentially displays each obtained in-vivo image on a display. Such image display device may set an extracting condition of the image to be displayed in advance, extract plural in-vivo images to be displayed from the in-vivo image group based on the set extracting condition, and display a list of such plural extracted in-vivo images on a screen. Medical staffs (hereinafter referred to as users) such as doctors and nurses can observe each in-vivo image displayed on such image display device, and observe (diagnose) the inside of the organ of the subject by observing the in-vivo images.

Japanese Laid-open Patent Publication No. 2007-222657 discloses an image display device for displaying an in-vivo image group of a subject, in which plural summarized graphical presentations of data stream and an image stream captured by a capsule endoscope are displayed. Japanese Laid-open Patent Publication No. 2007-519440 discloses a system that divides an image stream captured by a capsule endoscope into plural image streams and displays the plural image streams at substantially the same time.

However, in the conventional art described above, some in-vivo images are difficult to check, which are included in the series of in-vivo images captured by the capsule endoscope but not extracted to be displayed in a list, i.e., information on latent in-vivo images between the extracted in-vivo images displayed in a list on the screen. Thus, in the conventional art, the in-vivo images that can be viewed by a user are limited. As a result, the user cannot diagnose a site and neighboring sites, to which an attention is being paid, from various perspectives.

The present invention has been made in view of the above, and an object of the present invention is to provide an image display device overcoming at least some of the deficiencies of the prior art.

### DISCLOSURE OF INVENTION

An image display device according to an aspect of the present invention includes the features of claim 1.

The above and other features, advantages and technical and industrial significance of this invention will be better understood by reading the following detailed description of presently preferred embodiments of the invention, when considered in connection with the accompanying drawings.

### BRIEF DESCRIPTION OF DRAWINGS

FIG. 1 is a schematic view illustrating one configuration example of an in-vivo information acquiring system including an image display device according to a first Example of the present invention;
FIG. 2 is a block diagram schematically illustrating one configuration example of the image display device according to the first example of the present invention;
FIG. 3 is a flowchart illustrating a processing procedure of the image display device for displaying a mark of a characteristic image and displaying a plurality of in-vivo images in a list;
FIG. 4 is a schematic view illustrating a list-display of the in-vivo images by the image display device according to the first example of the present invention;
FIG. 5 is a schematic view illustrating a state in which the image display device additionally displays the characteristic image corresponding to the mark;
FIG. 6 is a block diagram schematically illustrating one configuration example of an image display device according to a second example of the present invention;
FIG. 7 is a flowchart illustrating a processing procedure of the image display device when displaying additional information indicating the number of latent images and displaying a plurality of in-vivo images in a list;
FIG. 8 is a schematic view illustrating a list-display of the in-vivo images by the image display device according to the second example of the present invention;
FIG. 9 is a block diagram schematically illustrating one configuration example of an image display device according to a third example of the present invention;
FIG. 10 is a schematic view illustrating a list-display of in-vivo images by the image display device according to the third example of the present invention;
FIG. 11 is a schematic view illustrating one specific example of additional information indicating information of a latent image;
FIG. 12 is a schematic view illustrating another specific example of additional information indicating information of a latent image;
FIG. 13 is a schematic view illustrating a modification example of a list-display of the in-vivo images by the image display device according to the second example ;
FIG. 14 is a block diagram schematically illustrating one configuration example of an image display device according to a fourth example of the present invention;
FIG. 15 is a schematic view illustrating one specific example of a display of a display unit according to the fourth example of the present invention;
FIG. 16 is a flowchart illustrating a processing procedure of a control unit of the image display device until displaying latent images latently included between representative images in a list;
FIG. 17 is a schematic view specifically describing an operation of the image display device according to the fourth example of the present invention;
FIG. 18 is a block diagram schematically illustrating one configuration example of an image display device according to a first embodiment of the present invention;
FIG. 19 is a schematic view illustrating one specific example of a display of a display unit according to the first embodiment of the present invention;
FIG. 20 is a schematic view specifically describing the operation of the image display device according to the first embodiment of the present invention;
FIG. 21 is a block diagram schematically illustrating one configuration example of an image display device according to a fifth example;
FIG. 22 is a schematic view illustrating one specific example of a display of a display unit according to the fifth example;
FIG. 23 is a schematic view illustrating one specific example of a setup menu for setting an image to non-display;
FIG. 24 is a schematic view specifically describing a non-display setting operation of the image by the image display device according to the fifth example;
FIG. 25 is a block diagram schematically illustrating one configuration example of an image display device according to a sixth example;
FIG. 26 is a schematic view illustrating one specific example of a setup menu for setting an image as a display target;
FIG. 27 is a schematic view specifically describing an operation of the image display device according to the sixth example;
FIG. 28 is a block diagram schematically illustrating one configuration example of an image display device according to a seventh example;
FIG. 29 is a schematic view illustrating one specific example of a display of a display unit according to the seventh example;
FIG. 30 is a schematic view illustrating a display of the display unit of when illustrating a time position of each image using a digestive tract schematic image;
FIG. 31 is a schematic view illustrating one modification example of a detailed-display area displaying latent images latently included between representative images in a list; and
FIG. 32 is a schematic view illustrating another list display example of the latent image.

### BEST MODE(S) FOR CARRYING OUT THE INVENTION

Exemplary embodiments of an image display device, an image display method, and a recording medium recorded with an image display program according to the present invention will be hereinafter described in detail with reference to the drawings. A capsule endoscope will be described as one example of an endoscope in an in-vivo information acquiring system according to the present invention, but the present invention is not limited to such embodiment.

### First Example

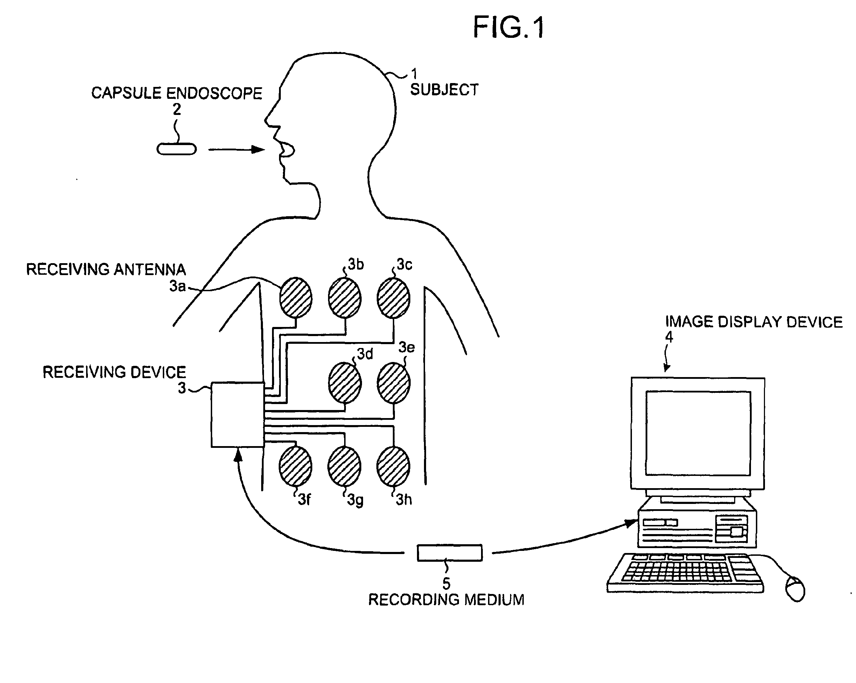

FIG. 1 is a schematic view illustrating one configuration example of an in-vivo information acquiring system including an image display device according to a first example. As illustrated in FIG. 1, the in-vivo information acquiring system according to the first example includes a capsule endoscope 2 for capturing an in-vivo image of a subject 1, a receiving device 3 for receiving an image signal wirelessly transmitted by the capsule endoscope 2, an image display device 4 for displaying the in-vivo image captured by the capsule endoscope 2, and a portable recording medium 5 for exchanging data between such receiving device 3 and the image display device 4.

The capsule endoscope 2 is a capsule-type endoscope device formed to a size that can be inserted to the inside of the organ of the subject 1, and has a capturing function and a wireless communication function inside a capsule-shaped casing. Specifically, the capsule endoscope 2 is inserted to the inside of the organ of the subject 1 by oral ingestion, etc. The capsule endoscope 2 inside the subject 1 then sequentially captures the in-vivo images in time-series and sequentially wirelessly transmits the image signals of the obtained in-vivo images to the external receiving device 3 while moving inside the organ by peristaltic motion, etc. The capsule endoscope 2 adds time data indicating the captured time of the in-vivo image to the image signal, and sequentially wirelessly transmits the image signal containing the image data of the in-vivo image and the time data. The capsule endoscope 2 sequentially repeats the capturing operation and the wirelessly transmitting operation of such in-vivo images during the period from when inserted to the inside of the subject 1 until excreted to the outside of the subject 1.

The receiving device 3 includes a plurality of receiving antennas 3a to 3h, where the wireless signal from the capsule endoscope 2 inside the subject 1 is received via at least one of the plurality of receiving antennas 3a. to 3h. The receiving device 3 performs a predetermined communication process such as demodulation process on the wireless signal received from such capsule endoscope 2, extracts the image signal contained in the wireless signal, and generates the in-vivo image of the subject 1 based on the extracted image signal. The receiving device 3 sequentially repeats such processes, and acquires the in-vivo image group of the subject 1 by the capsule endoscope 2. The receiving device 3 acquires the image data of the in-vivo image and also acquires the time data of the in-vivo image added to the image signal. The recording medium 5 is attached to the receiving device 3 in advance. The receiving device 3 stores each image data of the in-vivo image group of the subject 1 in the recording medium 5 and the time data such as the captured time of each in-vivo image in association with each other.

The receiving antennas 3a to 3h of such receiving device 3 may be dispersedly arranged on the body surface of the subject 1 as illustrated in FIG. 1, or may be arranged on a jacket to be worn by the subject 1. The number of receiving antennas of the receiving device 3 merely needs to be one or more, and is not particularly limited to eight.

The image display device 4 has a configuration similar to a workstation and acquires various types of data such as the in-vivo image group of the subject 1, and displays the various types of acquired data such as the in-vivo image. Specifically, the recording medium 5 detached from the above-described receiving device 3 is attached to the image display device 4, so that the image display device 4 can take the saved data of the recording medium 5. The image display device 4 thereby acquires various types of data such as the in-vivo image group of the subject 1 captured by the capsule endoscope 2. Such image display device 4 still-image displays or moving-image displays each in-vivo image in the in-vivo image group acquired in the above manner, or displays a list of a plurality of in-vivo images extracted from the in-vivo image group. The medical staffs such as doctors and nurses can observe each in-vivo image displayed on the image display device 4, observe the inside of the organ of the subject 1 by observing each in-vivo image, and consequently, make a diagnosis on the subject 1.

The recording medium 5 is a portable recording medium for exchanging data between the above-described receiving device 3 and the image display device 4. Specifically, the recording medium 5 is removable with respect to the receiving device 3 and the image display device 4, and has a structure of enabling output and recording of data when attached with respect to the receiving device 3 and the image display device 4. When attached to the receiving device 3, the recording medium 5 records various type of data such as the in-vivo image group, which the receiving device 3 has received from the capsule endoscope 2. The recording medium 5 stored with such various types of data is then detached from the receiving device 3 and attached to the image display device 4. In this case, the saved data of such recording medium 5 is taken into the image display device 4.

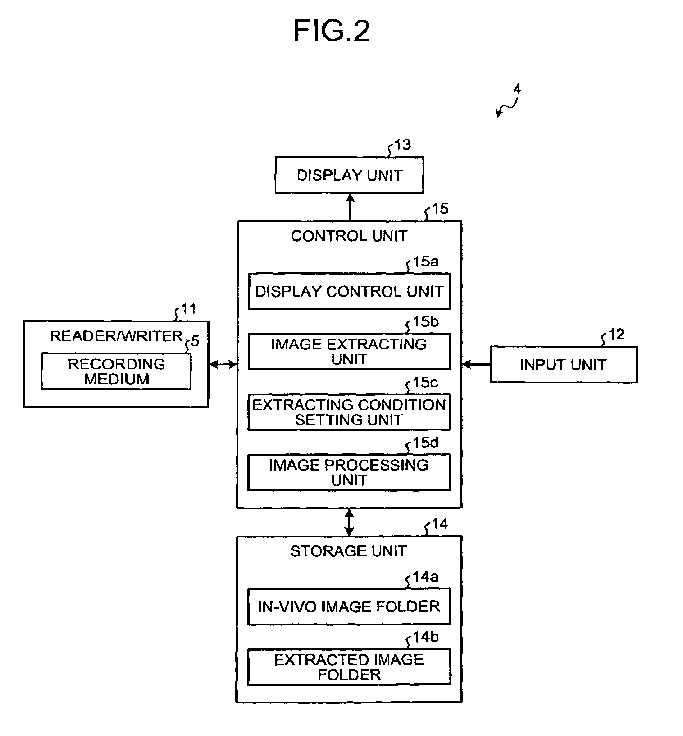

The configuration of the image display device 4 according to the first embodiment of the present invention will now be described in detail. FIG. 2 is a block diagram schematically illustrating one configuration example of the image display device according to the first embodiment of the present invention. As illustrated in FIG. 2, the image display device 4 according to the first embodiment includes a reader/writer 11 for taking in the saved data from the above-described recording medium 5, an input unit 12 for inputting various types of information, a display unit 13 for displaying the in-vivo images, etc., of the subject 1, a storage unit 14 for storing various types of data, etc., taken by the reader/writer 11, and a control unit 15 for controlling each unit of such image display device 4.

The reader/writer 11 functions as an image acquiring unit for acquiring the in-vivo image group of the subject 1 captured by the above-described capsule endoscope 2. Specifically, the reader/writer 11 is removably attached with the recording medium 5 detached from the above-described receiving device 3, and takes in the saved data from the recording medium 5 under the control of the control unit 15. The reader/writer 11 acquires various types of data such as the image data and the time data of the in-vivo image group of the subject 1 and transfers the various types of acquired data to the control unit 15. The in-vivo image group acquired by such reader/writer 11 is a series of images of the inside of the organ of the subject 1 captured by the above-described capsule endoscope 2 in time-series. If the initialized recording medium 5 is attached, the reader/writer 11 writes the data write-instructed by the control unit 15 on the recording medium 5. In this case, the reader/writer 11 writes specific data such as patient name, patient ID, examination date, and examination ID of the subject 1 on the recording medium 5.

The input unit 12 is constructed using an input device such as keyboard and mouse, and inputs various types of information to the control unit 15 in response to the input operation by the user. The various types of information input to the control unit 15 by such input unit 12 includes various types of instructing information to instruct the control unit 15, specific data of the subject 1, information of the image extracting condition for extracting the in-vivo images to be displayed in a list, and the like.

The display unit 13 is constructed using a display device capable of displaying images, such as CRT display and liquid crystal display, and displays various types of information and various types of data display-instructed by the control unit 15. Specifically, the display unit 13 displays the in-vivo image group of the subject 1 captured by the above-described capsule endoscope 2, the specific data of the subject 1, the input information input by the input unit 12, various types of GUI (Graphical User Interface) for input operation or display operation, and the like. More specifically, the display unit 13 still-image displays or moving-image displays each in-vivo image in the in-vivo image group of the subject 1 in time-series order or in reverse time-series order under the control of the control unit 15. The display unit 13 displays a plurality of in-vivo images extracted from the in-vivo image group of the subject 1 in a list under the control of the control unit 15. At the same time, the display unit 13 displays information on in-vivo image not extracted to be displayed in a list of the in-vivo image group of the subject 1, that is, information on some in-vivo images (hereinafter sometimes referred to as latent images) that are latently included between the images of the plurality of in-vivo images.

The storage unit 14 is constructed using a rewritable non-volatile storage medium such as EEPROM and hard disc. The storage unit 14 saves various types of data write-instructed by the control unit 15, and transmits the saved data read-instructed by the control unit 15 to the control unit 15. Specifically, the storage unit 14 stores various types of data and various types of information such as the input information input by the input unit 12 such as the specific data of the subject 1, the acquired data acquired by the reader/writer 11 such as the in-vivo image group of the subject 1, and the image extracting condition for extracting the in-vivo images to be displayed in a list, under the control of the control unit 15. The storage unit 14 includes an in-vivo image folder 14a, which is a storage area for storing all the acquired in-vivo images, and an extracted image folder 14b, which is a storage area for storing the in-vivo images extracted from the in-vivo image group of the in-vivo image folder 14a. The storage unit 14 stores the in-vivo image group of the above-described subject 1 in the in-vivo image folder 14a, and stores a plurality of in-vivo images extracted to be displayed in a list from the in-vivo image group in the extracted image folder 14b. The storage unit 14 may not has folders, such as in-vivo image folder 14a and the extracted image folder 14b, to store each in-vivo image data of the subject 1. In this case, the storage unit 14 may add identifiable additional information such as a flag to each in-vivo image extracted to be displayed in a list from the in-vivo image group of the subject 1, and store each in-vivo images to be displayed in a list so as to be distinguishable from the in-vivo image not to be displayed in a list.

The control unit 15 is constructed using a storage unit for storing programs, etc., for realizing the functions of the image display device 4, a CPU for executing the program in the storage unit, and the like. The control unit 15 controls each operation of the reader/writer 11, the input unit 12, the display unit 13, and the storage unit 14, which are components of the image display device 4, and controls input/output of signals between such components. Specifically, the control unit 15 controls the reader/writer 11 to take in the saved data from the recording medium 5 in accordance with the instructing information input by the input unit 12, and controls the storage unit 14 to store the taken saved data. The control unit 15 also controls the reader/writer 11 to save the specific data of the subject 1, etc., input by the input unit 12 to the recording medium 5.

The control unit 15 includes a display control unit 15a for controlling the display operation of the display unit 13, an image extracting unit 15b for extracting the in-vivo images to be displayed in a list, an extracting condition setting unit 15c for setting the image extracting condition, and an image processing unit 15d for performing various types of processes on each in-vivo image of the in-vivo image group.

The display control unit 15a controls the display unit 13 so as to still-image display or moving-image display each in-vivo image of the in-vivo image group of the subject 1 in accordance with the instructing information input by the input unit 12. In this case, the display control unit 15a reads out the in-vivo image group of the subject 1 from the in-vivo image folder 14a of the storage unit 14, and controls the display unit 13 so as to still-image display or moving-image display each in-vivo image of the read in-vivo image group in time-series order or reverse time-series order. The display control unit 15a sets, in advance, the displaying number of in-vivo images to display on one screen in accordance with information input by the input unit 12. The display control unit 15a controls the display unit 13 so as to display in a list a plurality of in-vivo images of the in-vivo image group of the subject 1 together with the information of the latent images that are latently included between the images of the plurality of in-vivo images displayed in a list in accordance with the instructing information input by the input unit 12. In this case, the display control unit 15a reads out the plurality of in-vivo images from the extracted image folder 14b of the storage unit 14 by the preset displaying number. The display control unit 15a controls the display unit 13 so as to add a mark illustrating the information of such latent image (specifically, type of characteristic image of the latent image, etc.) on a position between the images of the plurality of in-vivo images and display the plurality of in-vivo images in a list. When a desired mark is selected by the operation of the input unit 12 by the user from the marks between such images, the display control unit 15a controls the display unit 13 so as to additionally display the latent image corresponding to the selected mark. The plurality of in-vivo images saved in the extracted image folder 14b is extracted from the in-vivo image group of the subject 1 by the image extracting unit 15b.

The display control unit 15a determines whether or not the plurality of in-vivo images to be displayed in a list on the display unit 13 includes a characteristic image, and controls the display unit 13 to add a mark indicating the type of characteristic image to the characteristic image in the plurality of in-vivo images to be displayed in a list, if the characteristic image exists. The display control unit 15a sets display and non-display (hide) of such mark in accordance with the instructing information input by the input unit 12.

The above-described characteristic image is a characteristic in-vivo image, to which medical staffs such as doctors and nurses pay attention, and may be an in-vivo image assumed to be the bleeding part, an in-vivo image capturing the lesion part, an in-vivo image illustrating foreign particle such as a clip, an in-vivo image of the seam of the organs such as pylorus, an in-vivo image selected in accordance with the input information of the input unit 12 from in-vivo images displayed on the display unit 13, and the like. The above-described mark has a different color or shape for every type of such characteristic image.

The image extracting unit 15b extracts a plurality of in-vivo images to be displayed in a list from the in-vivo image group of the subject 1 based on the image extracting condition set by the extracting condition setting unit 15c. In this case, the image extracting unit 15b extracts the in-vivo image that conforms with the image extracting condition set by the extracting condition setting unit 15c from the in-vivo image group of the subject 1 in the in-vivo image folder 14a. The in-vivo image extracted by such image extracting unit 15b is saved in the extracted image folder 14b of the storage unit 14. When the extracting condition setting unit 15c changes the image extracting condition, the image extracting unit 15b re-extracts the in-vivo image that conforms with the changed image extracting condition from the in-vivo image group of the subject 1 every time.

The extracting condition setting unit 15c sets the image extracting condition for the above-described image extracting unit 15b to extract the in-vivo images to be displayed in a list from the in-vivo image group of the subject 1. The extracting condition setting unit 15c changes the image extracting condition in accordance with the input information of the input unit 12. The image extracting condition is saved in the storage unit 14 under the control of the control unit 15, and is appropriately read out by the control unit 15. The image extracting condition set by such extracting condition setting unit 15c is hierarchically changeable, and may be, for example, a threshold value of similarity between the in-vivo images that are successive in time-series, and the like.

The image processing unit 15d performs a predetermined image process on each in-vivo image in the in-vivo image group of the subject 1, and calculates the information necessary for the extracting process of the in-vivo image by the above-described image extracting unit 15b. Specifically, the image processing unit 15d reads out the in-vivo image group of the subject 1 saved in the in-vivo image folder 14a of the storage unit 14, and extracts the color information of each in-vivo image in the in-vivo image group. The image processing unit 15d calculates, for every image, the similarity between the in-vivo images successive in time-series in the in-vivo image group based on the color information of each in-vivo image. The image processing unit 15d may calculate the movement vector between the adjacent images in the in-vivo image group, and calculate the similarity between such in-vivo images for every image based on the calculated movement vector of each in-vivo image. The image processing unit 15d then associates each similarity calculated for every image with each in-vivo image in the in-vivo image group. The control unit 15 saves each similarity and each in-vivo image in the storage unit 14 in association with each other. The similarity of each in-vivo image saved in such storage unit 14 is appropriately read out by the control unit 15, and used when the above-described image extracting unit 15b extracts the in-vivo images to be displayed in a list.

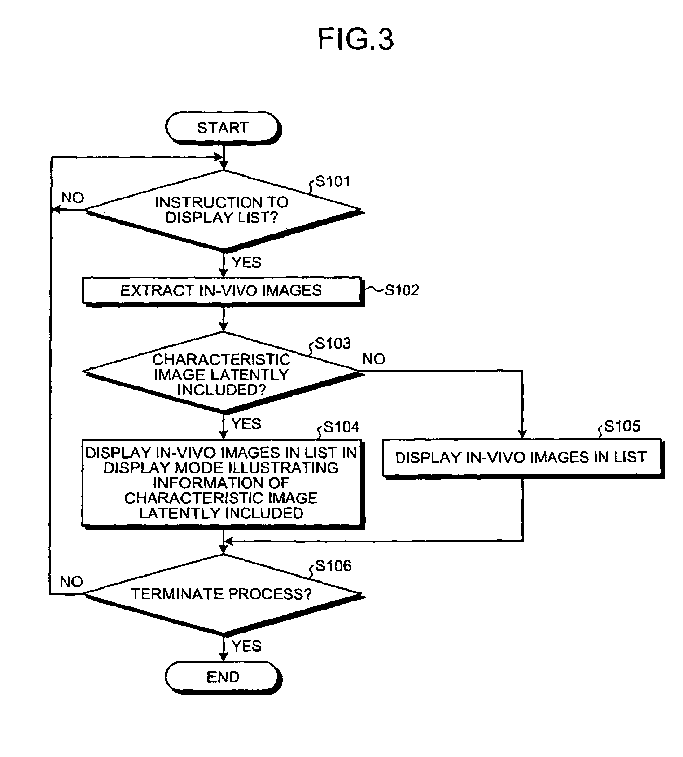

The operation of the image display device 4 according to the first example will now be described. FIG. 3 is a flowchart illustrating the processing procedures of the image display device for displaying the mark of the characteristic image and displaying a plurality of in-vivo images in a list. The image display device 4 according to the first example extracts a plurality of in-vivo images to be displayed in a list from the acquired in-vivo image group of the subject 1, and displays the plurality of in-vivo images in a list by the preset displaying number. In this case, if a characteristic image is contained in the latent image that latently exists between the images of the plurality of in-vivo images, the image display device 4 displays the mark of the characteristic image as the information of such latent image.

Specifically, as illustrated in FIG. 3, the control unit 15 of the image display device 4 according to the first example first determines the presence of an instruction to display the in-vivo images in a list (step S101). In step S101, the control unit 15 determines that the instruction to display the in-vivo images in a list is made if input with the instructing information to display a list by the input unit 12, and determines that the instruction to display the in-vivo images in a list is not made if not input with the instructing information to display a list. When determining that the instruction to display the in-vivo images in a list is not made (step S101, No), the control unit 15 repeats step S101 until the instructing information to display a list is input by the input unit 12.

When determining that the instruction to display the in-vivo images in a list is made in step S101 (step S101, Yes), the control unit 15 extracts the in-vivo images to display in a list from the in-vivo image group acquired in advance (step S102).

In step S102, the image extracting unit 15b extracts a plurality of in-vivo images that conform with the image extracting conditions set in advance by the extracting condition setting unit 15c from the in-vivo image group of the subject 1 saved in the in-vivo image folder 14a of the storage unit 14. In this case, the image extracting unit 15b compares the similarity of each in-vivo image in the in-vivo image group with the image extracting condition, and extracts the plurality of in-vivo images based on the comparison result. The similarity of each in-vivo image is calculated in advance by the image processing unit 15d, as described above. The control unit 15 controls the storage unit 14 so as to store the extracted plurality of in-vivo images in the extracted image folder 14b.

The control unit 15 then determines whether or not a characteristic image latently exists between the images of the plurality of in-vivo images to be displayed in a list (step S103). In step S103, the display control unit 15a reads out the in-vivo image other than the in-vivo images to be displayed in a list from the in-vivo image group of the subject 1 saved in the in-vivo image folder 14a, that is, the latent image that latently exists between the images of the plurality of in-vivo images to be displayed in a list from the storage unit 14. If the characteristic image is the in-vivo image including a characteristic site such as a bleeding part or a lesion part as the subject, such characteristic image contains color information (red, for example) inherent to the bleeding part or the lesion part, or the like. If the characteristic image is the in-vivo image selected in accordance with the input information of the input unit 12, such characteristic image is added with a flag indicating that it is the in-vivo image selected by the user from each in-vivo image displayed on the display unit 13. The display control unit 15a determines whether or not the latent image is the characteristic image with the color information or the flag of the latent image read out from such storage unit 14. The display control unit 15a repeatedly executes the determination process of such characteristic image for all the latent images included in the storage unit 14.

If at least one of all the latent images is a characteristic image, the control unit 15 determines that the characteristic image latently exists between the images of the plurality of in-vivo images to be displayed in a list (step S103, Yes), and displays the in-vivo images in a list on the display unit 13 in a display mode illustrating the information of the latently existing characteristic image (step S104).

In step S104, the display control unit 15a reads out the plurality of in-vivo images extracted by the image extracting unit 15b in step S102 from the extracted image folder 14b of the storage unit 14. The display control unit 15a controls the display unit 13 so as to display the plurality of in-vivo images in a list by the displaying number of in-vivo images in one screen set in advance. Furthermore, the display control unit 15a controls the display unit 13 so as to add a mark indicating the type of characteristic image to a position between the images where the characteristic image determined in step S103 latently exists.

The display unit 13 displays in a list the plurality of in-vivo images to be displayed in a list, and displays the mark indicating the type of the latently existing characteristic image to a position between the images where the characteristic image latently exists under the control of the display control unit 15a.

If none of the latent images in the storage unit 14 is the characteristic image in step S103, the control unit 15 determines that the characteristic image does not latently exist between the images of the plurality of in-vivo images to be displayed in a list (step S103, No). In this case, the control unit 15 displays the plurality of in-vivo images extracted in step S102 in a list on the display unit 13 (step S105). In step S105, the display control unit 15a reads out the plurality of in-vivo images extracted by the image extracting unit 15b in step S102 from the extracted image folder 14b of the storage unit 14. The display control unit 15a then controls the display unit 13 so as to display the plurality of in-vivo images in a list by the displaying number of in-vivo images in one screen set in advance.

Thereafter, the control unit 15 determines whether or not the list-displaying process of the in-vivo images is to be terminated (step S106), and determines to terminate the process in accordance with the input instructing information if the instructing information to terminate the process is input by the input unit 12 (step S106, Yes), and terminates the process. If the instructing information to terminate the process is not input in step S106, the control unit 15 determines not to terminate the process (step S106, No), returns to step S101, and repeats the processing procedures of step S101 and subsequent steps.

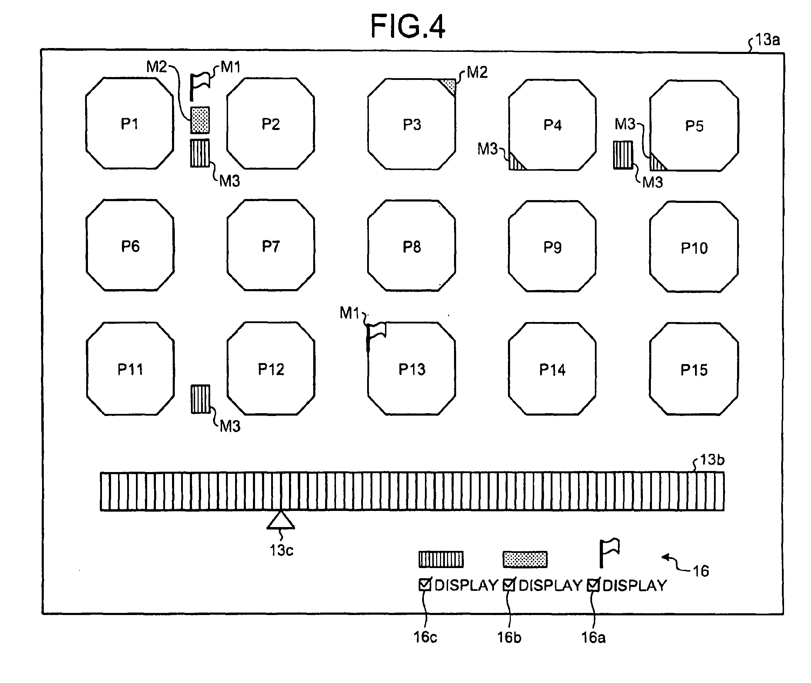

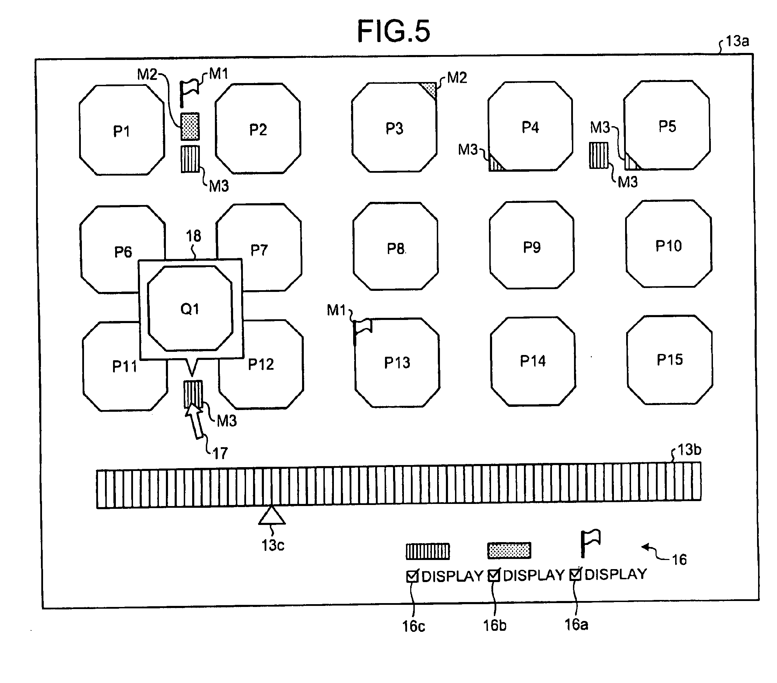

The operation of the image display device 4 of when displaying the in-vivo images in a list in the display mode illustrating the information of the characteristic image that latently exists between the images will be specifically described with the displaying number of in-vivo images to be displayed in a list in one screen as fifteen. FIG. 4 is a schematic view illustrating a list-display mode of the in-vivo images by the image display device according to the first example. FIG. 5 is a schematic view illustrating a state in which the image display device additionally displays the characteristic image corresponding to the mark.

In the image display device 4 in which the instructing information to display the in-vivo images in a list is input by the input unit 12, the control unit 15 determines that the instruction to display the in-vivo images in a list is made in accordance with the instructing information to display a list input by the input unit 12. In such state, the image extracting unit 15b extracts the in-vivo images P1 to P15 that conform with the image extracting conditions set by the extracting condition setting unit 15c from the in-vivo image group of the subject 1 saved in the in-vivo image folder 14a of the storage unit 14. The control unit 15 controls the storage unit 14 so as to store the plurality of in-vivo images P1 to P15 in the extracted image folder 14b as the in-vivo images to be displayed in a list.

The display control unit 15a then determines whether or not the characteristic image latently exists between the images of the plurality of in-vivo images P1 to P15 to be displayed in a list. In this case, the display control unit 15a reads out latent images that latently exist between the images of the plurality of in-vivo images P1 to P15 to be displayed in a list from the in-vivo image group of the subject 1 saved in the in-vivo image folder 14a.

The display control unit 15a determines whether or not the latent image is the characteristic image based on the color information or the additional information such as flag of the read latent image. If the latent image is the characteristic image, the display control unit 15a also determines the type of the characteristic image based own the color information or the additional information of the latent image. The display control unit 15a repeatedly executes the determination process and the type determination process of such characteristic image for all the latent images that latently exist between the images of the in-vivo images P1 to P15. The display control unit 15a may determine whether or not the latent image is the characteristic image based on the color information or the additional information of the latent image read out from the storage unit 14, and determine the type of such characteristic image. Alternatively, the display control unit 15a may determine whether or not each in-vivo image is the characteristic image and determine the type of the characteristic image in real time at the timing when the data of the in-vivo image group is acquired from the reader/writer 11.

Thereafter, the display control unit 15a associates a mark, which is different for every type of characteristic image, with each characteristic image of all the latent images between the images of the in-vivo images P1 to P15. The display control unit 15a controls the display unit 13 so as to display in a list the plurality of in-vivo images P1 to P15 to be displayed in a list. The display control unit 15a also controls the display unit 13 so as to add the mark indicating the type of characteristic image to a position between the plurality of in-vivo images P1 to P15 where the characteristic image latently exists. In this case, the display control unit 15a controls the display unit 13 so as to add the mark, which is associated with the characteristic image that latently exists between the images, to a position between the images. The display control unit 15a repeatedly executes the control of the display unit 13 for every characteristic image that latently exists between the images of the plurality of in-vivo images P1 to P15.

The display unit 13 displays in a list the in-vivo images P1 to P15 to be displayed in a list, and displays the mark of the characteristic image to a position between the plurality of in-vivo images P1 to P15 where the characteristic image latently exists under the control of the display control unit 15a. Specifically, as illustrated in FIG. 4, the display unit 13 displays the plurality of in-vivo images P1. to P15 in a list in a list-display window 13a of the in-vivo image. The display unit 13 also displays marks M1 to M3 at a position between the images of the in-vivo images P1, P2 where the characteristic image latently exists, displays the mark M3 at a position between the images of the in-vivo images P4, P5, and displays the mark M3 at a position between the images of the in-vivo images P11, P12.

As illustrated in FIG. 4, such marks M1 to M3 indicate the type of characteristic images that are different from each other. The mark M1 is a mark indicating the characteristic image selected in accordance with the input information of the input unit 12, that is, the in-vivo image selected by the user from each in-vivo image displayed on the display unit 13 (hereinafter sometimes referred to as pickup image). The mark M2 is a mark indicating the in-vivo image including the bleeding part as the subject. The mark M3 is a mark indicating the in-vivo image including the lesion part as the subject.

The above-described display control unit 15a can switch the setting of display and non-display of the marks M1 to M3 of such characteristic image in accordance with the input information of the input unit 12. Specifically, as illustrated in FIG. 4, the display unit 13 displays a setting GUI 16 for setting the display or the non-display of the marks M1 to M3 in the list-display window 13a. The setting GUI 16 includes check boxes 16a to 16c for switching the setting of display and non-display of the marks M1 to M3 of the characteristic image. The check box 16a is for switching the setting of display and non-display of the mark M1 of the pickup image of the characteristic images. The check box 16b is for switching the setting of display and non-display of the mark M2 of the in-vivo image of the bleeding part of the characteristic images. The check box 16c is for switching the setting of display and non-display of the mark M3 of the in-vivo image of the lesion part of the characteristic images.

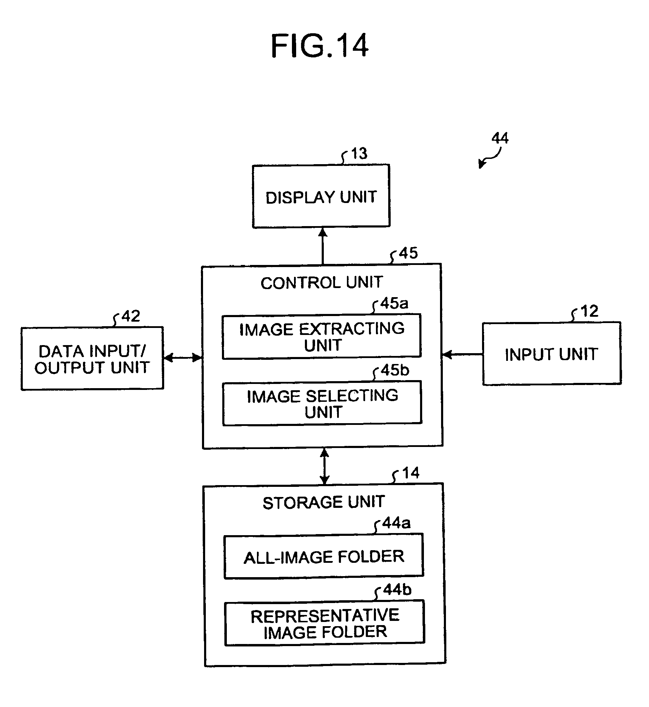

The input unit 12 inputs instructing information to the control unit 15 to display the mark M1 when the check box 16a is changed to the ON state by the click operation of the check box 16a, and inputs instructing information to the control unit 15 to hide the mark M1 when the check box 16a is changed to the OFF state. The input unit 12 inputs instructing information to the control unit 15 to display the mark M2 when the check box 16b is changed to the ON state by the click operation of the check box 16b, and inputs instructing information to the control unit 15 to hide the mark M2 when the check box 16a is changed to the OFF state. The input unit 12 inputs instructing information to the control unit 15 to display the mark M3 when the check box 16c is changed to the ON state by the click operation of the check box 16c, and inputs instructing information to the control unit 15 to hide the mark M3 when the check box 16a is changed to the OFF state. The check boxes 16a to 16c in the ON state are illustrated in FIG. 14.

The display control unit 15a sets each mark M1 to M3 to the display state or the non-display state based on instructing information of instructing the display setting or the non-display setting of the marks M1 to M3. The display unit 13 appropriately displays the marks M1 to M3 between the images of the in-vivo images P1 to P15 under the control of such display control unit 15a. In FIG. 4, all the marks M1 to M3 are set to the display state, and the display unit 13 appropriately displays the marks M1 to M3 between the images of the in-vivo images P1, P2, between the images of the in-vivo images P4, P5, and between the images of the in-vivo images P11, P12, as described above.

The user such as a doctor or a nurse can easily observe the plurality of in-vivo images P1 to P15 extracted based on the desired image extracting conditions from the in-vivo image group of the subject 1, and can easily recognize whether a characteristic image of any type latently exists between the images of the plurality of in-vivo images P1 to P15 by visually recognizing such list-display window 13a. The user can thus easily determine the relevance of the characteristic image latently existing between such images and the list-displayed in-vivo images P1 to P15, and can easily recognize the positional relationship of the plurality of in-vivo images P1 to P15 and the characteristic image illustrated by one of the marks M1 to M3. As a result, the target in-vivo image that is useful in observation and diagnosis of the inside of the organ of the subject 1 can be easily found.

The display control unit 15a determines whether or not the characteristic image is included in the plurality of in-vivo images P1 to P15 based on the color information or the additional information such as a flag of each in-vivo image of the plurality of in-vivo images P1 to P15 to be displayed in a list. If the characteristic image is included in the in-vivo images P1 to P15, the display control unit 15a controls the display unit 13 so as to add a mark indicating the type of characteristic image to the characteristic image of the in-vivo images P1 to P15. For instance, as illustrated in FIG. 4, the display unit 13 adds the mark M1 to the in-vivo image P13 which is the pickup image, adds the mark M2 to the in-vivo image P3 of the bleeding part, and adds the mark M3 to the in-vivo images P4, P5 of the lesion part under the control of such display control unit 15a. In this case, the display unit 13 appropriately displays the marks M1 to M3 in the vicinity of such in-vivo images P3, P4, P5, P13 which are the characteristic images (e.g., near the corner of the image).

The user such as a doctor or a nurse can easily determine that the in-vivo image P3 is the in-vivo image of the bleeding part by visually recognizing the mark M2 added to such in-vivo image P3. Similarly, the user can easily determine that the in-vivo images P4, P5 are the in-vivo images of the lesion part by visually recognizing the mark M3 added to such in-vivo images P4, P5 and can easily determine that the in-vivo image P13 is the pickup image by visually recognizing the mark M1 added to such in-vivo image P13.

When a desired mark is selected by the user from the marks M1 to M3 added to a position between the images of the plurality of in-vivo images P1 to P15 to be displayed in a list, the display control unit 15a additionally displays the latent image, which is a characteristic image corresponding to the selected mark, on the display unit 13. In other words, the input unit 12 inputs selecting information of selecting one of the marks M1 to M3 displayed between such images to the control unit 15 in response to the input operation by the user. The display control unit 15a controls the display unit 13 so as to additionally display the latent image corresponding to the mark selected by the selecting information.

Specifically, as illustrated in FIG. 5, for example, the input unit 12 inputs the selecting information of the mark M3 to the.control unit 15 in response to the click operation performed by placing a mouse cursor 17 on the mark M3 added to a position between the images of the in-vivo images P11, P12. The display control unit 15a controls the display unit 13 so as to additionally display the characteristic image Q1 corresponding to the mark selected by such selecting information, that is, the mark M3 between the images of the in-vivo images P11, P12. The display unit 13 additionally displays an image display window 18 including the characteristic image Q1 corresponding to the mark M3 under the control of such display control unit 15a. The display unit 13 pop-up displays the characteristic image Q1 latently existing between the images of the in-vivo images P11, P12.

The shape of such image display window 18 may be an arbitrary shape, but a balloon shape pointing the selected mark M3 is desirable, as illustrated in FIG. 5. The display unit 13 can easily display the relationship between the characteristic image Q1 in such image display window 18 and the mark M3.

The display unit 13 displays the list-display window 13a under the control of the display control unit 15a. As illustrated in FIGS. 4 and 5, such list-display window 13a is displayed with a time bar 13b and a slider 13c for indicating the time position of any one of the plurality of in-vivo images displayed in a list The time bar 13b is a graphic bar indicating the overall time position of the in-vivo image group of the subject 1. The slider 13c moves along such time bar 13b, and points out to the time position on the time bar 13b corresponding to the in-vivo image selected from the in-vivo images to be displayed in a list (in-vivo images P1 to P15 in FIGS. 4 and 5).

In FIGS. 4 and 5, the display control unit 15a sets the displaying number of in-vivo images to be displayed in a list in the list-display window 13a to fifteen, but the displaying number of in-vivo images in such list-display window 13a may be a desired number, and is not particularly limited to fifteen. In FIG. 5, the in-vivo images P1 to P15 of the plurality of in-vivo images to be displayed in a list are displayed, but the display unit 13 can display the remaining in-vivo images in a list by the scroll operation of the image, the movement operation of the slider 13c, or the setting change of the displaying number, or the like.

As described above, in the first example, whether or not a characteristic image latently exists between the images of the plurality of in-vivo images to be displayed in a list is determined, and if the characteristic image latently exists, the plurality of in-vivo images are displayed in a list with a mark indicating information such as type of the latently existing characteristic image added to a position between the images where the characteristic image latently exists. Thus, an image display device, an image display method, and an image display program enabling the information of the characteristic image that latently exists between the images of the plurality of in-vivo images to be checked while observing the plurality of in-vivo images displayed in a list on the screen can be realized.

Through the use of the image display device according to the first example, whether any characteristic image latently exists between the images of the plurality of in-vivo images can be easily recognized while observing the plurality of in-vivo images extracted based on the desired image extracting condition from the in-vivo image group of the subject. Thus, the relevance of the characteristic image that latently exists between such images and the plurality of in-vivo images displayed in a list can be easily determined, and the positional relationship of the plurality of in-vivo images and the characteristic image that latently exists between the images can be easily recognized. As a result, the target in-vivo image that is useful in observation and diagnosis of the inside of the organ of the subject can be easily found.

### Second example

A second example will now be described. In the first described above, the mark indicating the information of the characteristic image is added between the images, where the characteristic image latently exists, of each images of the plurality of in-vivo image to be displayed in a list, but in the second example, additional information indicating the number of latent images is added to a position between the images of the plurality of in-vivo images to be displayed in a list.

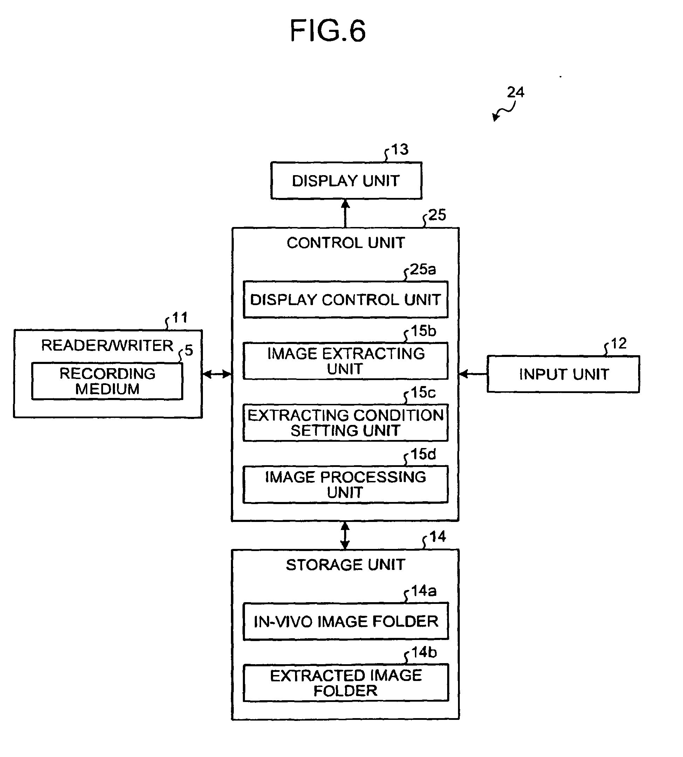

FIG. 6 is a block diagram schematically illustrating one configuration example of an image display device according to the second example. As illustrated in FIG. 6, an image display device 24 according to the second example includes a control unit 25 in place of the control unit 15 of the image display device 4 according to the first example described above. The control unit 25 includes a display control unit 25a in place of the display control unit 15a of the image display device 4 according to the first example described above. The in-vivo information acquiring system according to the second embodiment includes the image display device 24 in place of the image display device 4 of the in-vivo information acquiring system (see FIG. 1) according to the first example described above. Other configurations are the same as the first example, and the same reference numerals are denoted for the same components.

The control unit 25 includes the display control unit 25a in place of the display control unit 15a of the image display device 4 according to the first example, as described above. The control unit 25 has a function similar to the control unit 15 of the first embodiment described above other than the function of such display control unit 25a.

The display control unit 25a controls the display unit 13 so as to display the plurality of in-vivo images in a list with additional information indicating the information of the in-vivo image (i.e., latent image) that latently exists between the images of the plurality of in-vivo images to be displayed in a list added to a position between the images of the plurality of in-vivo images instead of the mark of the above-described characteristic image. Specifically, the display control unit 25a controls the display unit 13 so as to read out the plurality of in-vivo images from the extracted image folder 14b of the storage unit 14 in accordance with the instructing information input by the input unit 12, and display the plurality of in-vivo images in a list by the displaying number set in advance. Furthermore, the display control unit 25a controls the display unit 13 so as to add the additional information indicating the number of latent images between the images of the plurality of in-vivo images to a position between the images of the plurality of in-vivo images to be displayed in a list. The display control unit 25a has a function similar to the display control unit 15a of the image display device 4 according to the first embodiment described above other than the function of displaying the additional information indicating the number of latent images for every images on the display unit 13.

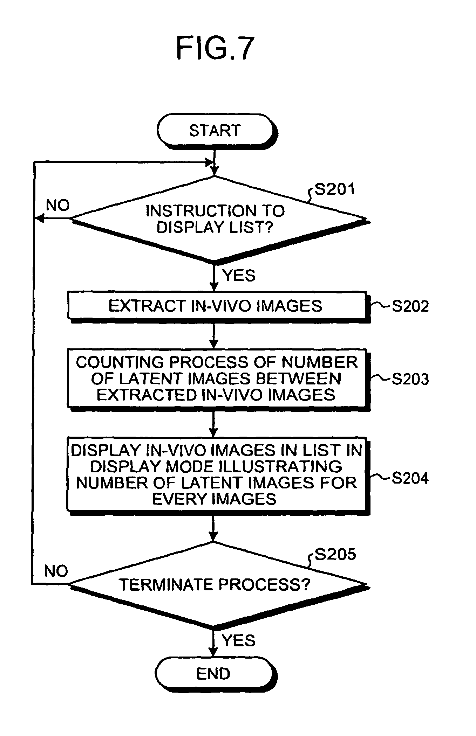

The operation of the image display device 24 according to the second example will now be described. FIG. 7 is a flowchart illustrating the processing procedures of the image display device when displaying the additional information indicating the number of latent images and displaying the plurality of in-vivo images in a list. The image display device 24 according to the second example extracts a plurality of in-vivo images to be displayed in a list from the acquired in-vivo image group of the subject 1, and displays the plurality of in-vivo images in a list by the displaying number set in advance. In this case, the image display device 24 displays the additional information indicating the number of latent images for every images, which is one example of information of the latent image, to a position between the images of the plurality of in-vivo images.

Specifically, as illustrated in FIG. 7, the control unit 25 of the image display device 24 according to the second embodiment first determines the presence of an instruction to display the in-vivo images in a list (step S201). In step S201, the control unit 25 determines the presence of the instruction to display the in-vivo images in a list similarly to step S101 illustrated in FIG. 3, and repeats step S201 until the instructing information of list-display is input by the input unit 12 when determined that the instruction to display the in-vivo images in a list is not made (step S201, No).

When determined that the instruction to display the in-vivo images in a list is made in step S201 (step S201, Yes), the control unit 25 extracts the in-vivo images to be displayed in a list from the in-vivo image group acquired in advance (step S202). In step S202, the image extracting unit 15b extracts a plurality of in-vivo images to be displayed in a list from the in-vivo image group of the subject 1, similarly to step S102 illustrated in FIG. 3. The control unit 25 controls the storage unit 14 so as to store the extracted plurality of in-vivo images in the extracted image folder 14b.

The control unit 25 then executes a counting process of the number of latent images between the images of the in-vivo images extracted in step S202 (step S203). In step S203, the display control unit 25a counts the number of remaining in-vivo images excluding the plurality of in-vivo images extracted to be displayed in a list by the image extracting unit 15b from the in-vivo image group of the subject 1 saved in the in-vivo image folder 14a of the storage unit 14 for every images of the plurality of in-vivo images. The number of remaining in-vivo images is the number of latent images that latently exist between the images of the plurality of in-vivo images to be displayed in a list. The display control unit 25a counts the number of latent images between the images of the plurality of in-vivo images to be displayed in a list by performing the counting process for each adjacent pair of images.

The control unit 25 displays the in-vivo images in a list on the display unit 13 in a display mode illustrating the number of latent images between the images (step S204). In step S204, the display control unit 25a reads out a plurality of in-vivo images extracted in step S202 by the image extracting unit 15b from the extracted image folder 14b of the storage unit 14. The display control unit 25a controls the display unit 13 so as to display a plurality of in-vivo images in a list by the displaying number of in-vivo images in one screen set in advance. The display control unit 25a controls the display unit 13 so as to add the additional information indicating the number of latent images between the images counting processed in step S203 to a position between the images of the plurality of in-vivo images.

The display unit 13 displays in a list a plurality of in-vivo images to be displayed in a list and displays the additional information indicating the number of latent images between the images to a position between the images of the plurality of in-vivo images under the control of such display control unit 25a.

Thereafter, the control unit 25 determines whether or not the list-displaying process of the in-vivo images is to be terminated (step S205), and terminates the process when determined to terminate the process (step S205, Yes), similarly to step S106 illustrated in FIG. 3. When determined not to terminate the process in step S205 (step S205, No), the control unit 25 returns to step S201, and repeats the processing procedures of step S01 and subsequent steps.

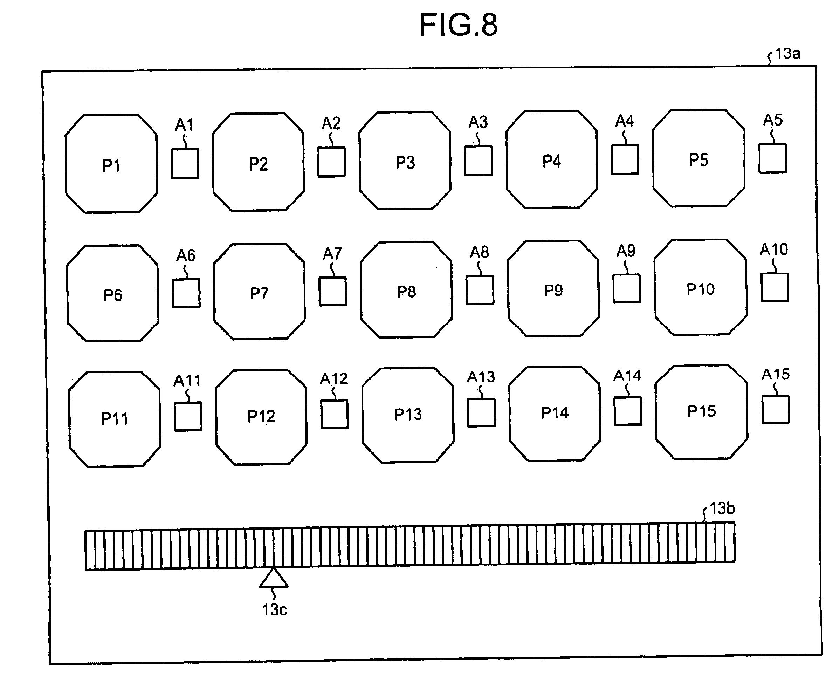

The operation of the image display device 24 in displaying the in-vivo images in a list in a display mode of indicating the information of the latent image that latently exists between the images will be specifically described with the displaying number of in-vivo images to be displayed in a list in one screen as fifteen. FIG. 8 is a schematic view illustrating a list-display mode of the in-vivo images by the image display device according to the second example.

In the image display device 24 in which the instructing information to display the in-vivo images in a list is input by the input unit 12, the control unit 25 determines that the instruction to display the in-vivo images in a list is made based on the instructing information to display a list input by the input unit 12. In such state, the image extracting unit 15b extracts the in-vivo images P1 to P15 that conform with the image extracting conditions by the extracting condition setting unit 15c from the in-vivo image group of the subject 1 saved in the in-vivo image folder 14a of the storage unit 14. The control unit 25 controls the storage unit 14 so as to store the plurality of in-vivo images P1 to P15 in the extracted image folder 14b as the in-vivo images to be displayed in a list.

The display control unit 25a then executes the counting process of the number of latent images for each adjacent pair of the images of the plurality of in-vivo images P1 to P15 to be displayed in a list. In this case, the display control unit 25a reads out the in-vivo image group of the subject 1 saved in the in-vivo image folder 14a of the storage unit 14. The display control unit 25a counts the number of latent images that latently exist between the images of the in-vivo images P1, P2, and counts the number of latent images that latently exist between the images of the in-vivo images P2, P3 of the read in-vivo image group. Such display control unit 25a repeatedly executes the counting process of the number of latent images similarly for images subsequent to the in-vivo image P3. The display control unit 25a counts the number of latent images for each adjacent pair the images of the plurality of in-vivo images P1 to P15.

If the above-described image extracting unit 15b extracts sixteen or more in-vivo images as the in-vivo images to be displayed in a list from the in-vivo image group of the subject 1, the display control unit 25a repeatedly executes the counting process of the number of latent images for the images subsequent to the fifteenth image to be displayed in a list (i.e., in-vivo image P15).

Thereafter, the display control unit 25a controls the display unit 13 so as to display in a list the plurality of in-vivo images P1 to P15 to be displayed in a list. The display control unit 25a also controls the display unit 13 so as to add the additional information indicating the number of latent images for every image to a position between each image of the plurality of in-vivo images P1 to P15. In this case, the display control unit 25a displays the counting process result of the number of latent images between the images of the in-vivo images P1 to p15 on the display unit 13 as the additional information. If the number of in-vivo images to be displayed in a list is greater than or equal to sixteen, the control of such display unit 13 is repeatedly executed by the display control unit 25a even on the images subsequent to the fifteenth image to be displayed in a list (i.e., in-vivo image P15).

The display unit 13 displays in a list the in-vivo images P1 to P15 to be displayed in a list and displays the additional information indicating the number of latent images between the images to a position between the images of the plurality of in-vivo images P1 to P15 under the control of such display control unit 25a. Specifically, as illustrated in FIG. 8, the display unit 13 displays the plurality of in-vivo images P1 to P15 in a list in the list-display window 13a of the in-vivo image. The display unit 13 also displays additional information A1 to A14 indicating the number of latent images between the images to a position between the images of the plurality of in-vivo images P1 to P15. The display unit 13 displays additional information A15 indicating the number of latent images between the images subsequent to the in-vivo image P15 on the side opposite to the additional information A14 at the side of the in-vivo image P15.

The additional information A1 to A15 are numerical value information indicating the counting process result of the number of latent images between the images by such display control unit 25a. Specifically, the additional information A1 is the numerical value information indicating the number of latent images that latently exist between the images of the in-vivo images P1, P2; the additional information A2 is the numerical value information indicating the number of latent images that latently exist between the images of the in-vivo images P2, P3; and the additional information A3 is the numerical value information indicating the number of latent images that latently exist between the images of the in-vivo images P3, P4. Similarly, the additional information A4 to A14 are numerical value information indicating the number of latent images between the images of the in-vivo images P4 to P15.

The additional information A15 is the numerical value information indicating the number of latent images that latently exist subsequent to the in-vivo image P15 that is the fifteenth image to be displayed in a list if the number of in-vivo images to be displayed in a list is fifteen. If the number of in-vivo images to be displayed in a list is greater than or equal to sixteen, the additional information A15 is the numerical value information indicating the number of latent images that latently exist between the images of the in-vivo image P15 and the sixteenth image to be displayed in a list.

The user such as a doctor or a nurse can easily observe the plurality of in-vivo images P1 to P15 extracted based on the desired image extracting conditions from the in-vivo image group of the subject 1, and can easily recognize how many in-vivo images latently exist between the images of the plurality of in-vivo images P1 to P15 by visually recognizing such list-display window 13a. The user can thus easily visually-recognize the interval of each image of such in-vivo images P1 to P15 displayed in a list, so that the positional relationship between the in-vivo images in the in-vivo images P1 to P15 can be easily grasped. As a result, each capturing site of the in-vivo images P1 to P15 displayed in a list can be easily determined, and the in-vivo images can be efficiently observed in a short period of time.

In FIG. 8, the display control unit 25a sets the displaying number of in-vivo images to display in a list in the list-display window 13a to fifteen, but the displaying number of in-vivo images in such list-display window 13a may be a desired number and is not particularly limited to fifteen. In FIG. 8, the in-vivo images P1 to P15 of the plurality of in-vivo images to be displayed in a list are displayed, but the display unit 13 can also display the remaining in-vivo images in a list by the scroll operation of the image, the movement operation of the slider 13c, or the setting change of the displaying number, or the like. In this case, the display control unit 25a controls the display unit 13 so as to add the additional information indicating the number of latent images between the images with respect to the images subsequent to the fifteenth image to be displayed (in-vivo image P15), similarly to a position between the images of the above-described in-vivo images P1 to p15. The display unit 13 displays the additional information indicating the number of latent images for every image to a position between the images subsequent to such in-vivo image P15 under the control of such display control unit 25a.

As described above, in the second embodiment of the present invention, the plurality of in-vivo images are displayed in a list with the additional information indicating the number, etc., of latent images that latently exist between the images of the plurality of in-vivo images to be displayed in a list added to a position between the images. Thus, the image display device, the image display method, an the image display program enabling the information of the latent image that latently exists between the images of the plurality of in-vivo images to be checked while observing the plurality of in-vivo images displayed in a list on the screen can be realized.

How many in-vivo images latently exist between the images of the plurality of in-vivo images can be easily recognized while observing the plurality of in-vivo images extracted based on the desired image extracting condition from the in-vivo image group of the subject by using the image display device according to the second example. Thus, each image interval of the plurality of in-vivo images displayed in a list can be visually recognized, and the positional relationship between the in-vivo images in the plurality of in-vivo images can be easily grasped. As a result, the in-vivo images can be efficiently observed in a short period of time while easily determining each capturing site of the plurality of in-vivo images.

### Third example

A third example will now be described. In the second example described above, the additional information indicating the number of latent images is added to a position between the images of the plurality of in-vivo images to be displayed in a list, but in the third example, the plurality of in-vivo images are displayed in a list with an inter-image distance of a plurality of in-vivo images to be displayed in a list changed depending on the number of latent images between the images.

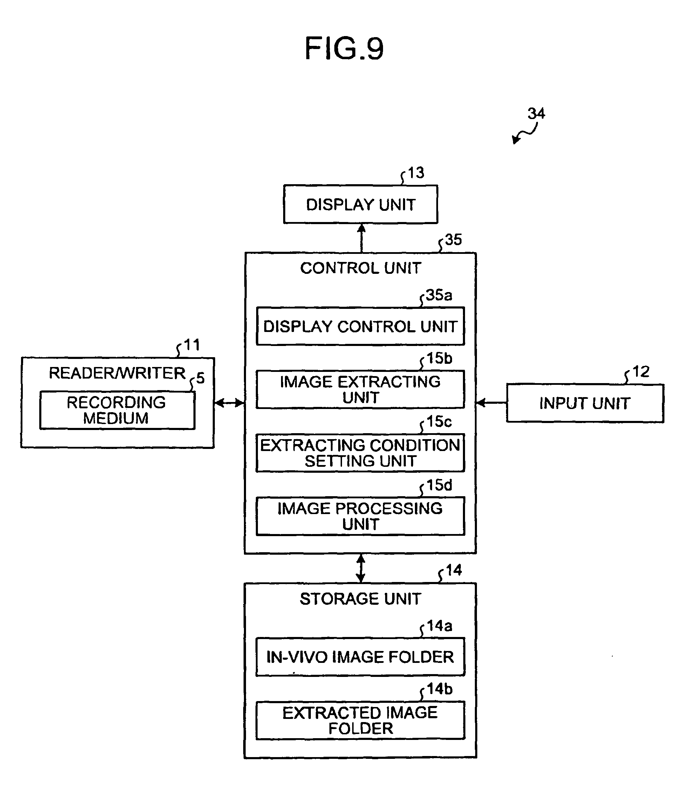

FIG. 9 is a block diagram schematically illustrating one configuration example of an image display device according to the third example. As illustrated in FIG. 9, an image display device 34 according to the third example includes a control unit 35 in place of the control unit 25 of the image display device 24 according to the second example described above. The control unit 35 includes a display control unit 35a in place of the display control unit 25a of the image display device 24 according to the second example described above. The in-vivo information acquiring system according to the third example includes the image display device 34 in place of the image display device 24 of the in-vivo information acquiring system according to the second example described above. Other configurations are the same as the second example, and the same reference numerals are denoted for the same components.

As described above, the control unit 35 includes the display control unit 35a in place of the display control unit 25a of the image display device 24 according to the second example described above. The control unit 35 has functions similar to the control unit 25 according to the second example described above other than the function of such display control unit 35a.

The display control unit 35a controls the display unit 13 so as to display a plurality of in-vivo images in a list by changing the inter-image distance of the plurality of in-vivo images to be displayed in a list depending on the number of latent images between the images instead of adding the additional information indicating the number of latent images between the above-described images. Specifically, the display control unit 35a reads out a plurality of in-vivo images from the extracted image folder 14b of the storage unit 14 in accordance with the instructing information input by the input unit 12. Similarly to the second example described above, the display control unit 35a counts the number of latent images between the images of the plurality of in-vivo images. The display control unit 35a controls the display unit 13 so as to display the plurality of in-vivo images in a list in a mode in which each inter-image distance is changed depending on the number of latent images of every image by the displaying number set in advance. The display control unit 35a has functions similar to the display control unit 25a of the image display device 24 according to the second example described above other than the function of displaying in a list on the display unit 13 each in-vivo image in a mode in which the inter-image distance is changed depending on the number of latent images between the images.

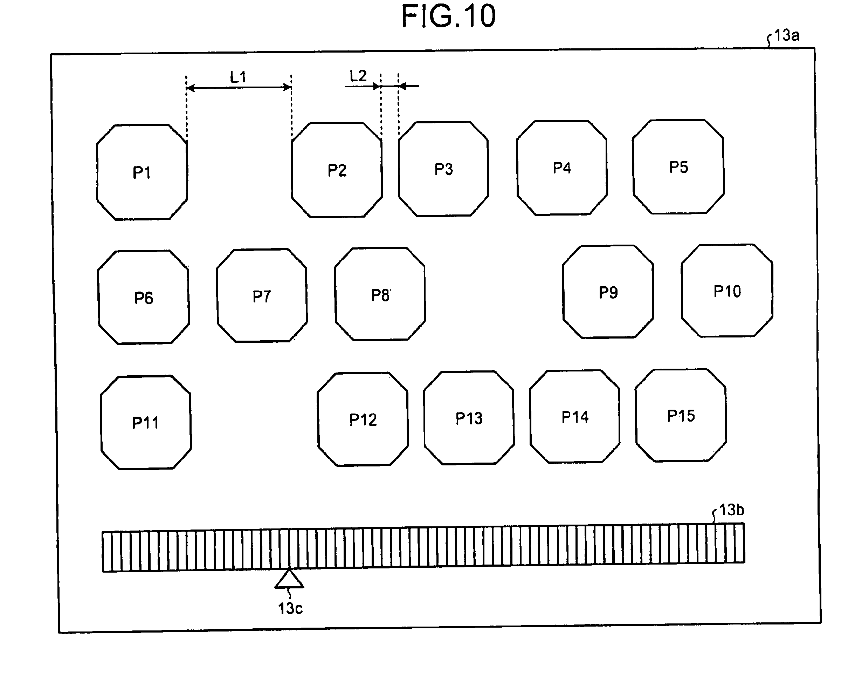

The operation of the image display device 34 according to the third example will now be described. FIG. 10 is a schematic view illustrating a list-display mode of the in-vivo image by the image display device according to the third example. The image display device 34 according to the third example operates similarly to the image display device 24 according to the second example described above other than the operation of displaying a plurality of in-vivo images in a list by changing the inter-image distance depending on the number of latent images for every image. In other words, the control unit 35 of the image display device 34 executes a processing procedure different from the second embodiment in step S204 of the steps S201 to S205 illustrated in FIG. 7. The control unit 35 executes a processing procedure similar to the second embodiment in the remaining steps S201 to S203 and step S205. Illustrating a case where the displaying number of in-vivo images to be displayed in a list on one screen is fifteen, the operation of the image display device 34 in step S204 will be described below.

In step S204 described above, the display control unit 35a controls the display unit 13 so as to display in a list in-vivo images P1 to P15 in a mode in which each inter-image distance of a plurality of in-vivo images P1 to P15 to be displayed in a list is changed depending on the number of latent images for every image. In this case, the display control unit 35a converts the number of latent images between the images to the inter-image distance, and controls the display unit 13 so as to display in a list the in-vivo images P1 to P15 spaced apart by the obtained inter-image distance. The inter-image distance controlled by such display control unit 35a increases with increase in the number of latent images between the images, and decreases with decrease in the number of latent images between the images. If the number of in-vivo images to be displayed in a list is greater than or equal to sixteen, the display control unit 35a repeatedly executes the control of such display unit 13 even for images subsequent to the fifteenth image to be displayed in a list (i.e., in-vivo image P15).

As illustrated in FIG. 10, the display unit 13 displays in a list the in-vivo images P1 to P15 to be displayed in a list in a mode of changing (increasing and decreasing) each inter-image distance depending on the number of latent images between the images under the control of such display control unit 35a. In this case, if the number of latent images between the images of the in-vivo images P1, P2 is greater than the number of latent images between the images of the in-vivo images P2, P3, the display unit 13 sets the inter-image distance L1 of the in-vivo images P1, P2 to be greater than the inter-image distance L2 of the in-vivo images P2, P3. Similarly, the display unit 13 sets the inter-image distance between the images where a large number of in-vivo images latently exist to be relatively large compared to other images, and sets the inter-image distance between the images where a small number of in-vivo images latently exist to be relatively small compared to other images between the images of the in-vivo images P1 to P15.

The user such as a doctor or a nurse can easily observe the plurality of in-vivo images P1 to P15 extracted based on the desired image extracting conditions from the in-vivo image group of the subject 1, and can easily compare the magnitude relationship of the number of in-vivo images (i.e., number of latent images) that latently exist between the images of the plurality of in-vivo images P1 to P15 by visually recognizing such list-display window 13a. The user can thus intuitively recognize each inter-image distance of such list-displayed in-vivo images P1 to P15 and can easily grasp the positional relationship between the in-vivo images of the in-vivo images P1 to P15. As a result, the in-vivo image can be efficiently observed in a short period of time while easily determining each capturing site of the list-displayed in-vivo images P1 to P15.

In FIG. 10, the display control unit 35a sets the displaying number of in-vivo images to be displayed in a list in the list-display window 13a to fifteen, but the displaying number of in-vivo images in such list-display window 13a may be a desired number, and is not particularly limited to fifteen. Furthermore, the in-vivo images P1 to P15 of the plurality of in-vivo images to be displayed in a list are displayed in FIG. 10, but the display unit 13 can display the remaining in-vivo images in a list by the scroll operation of the image, the movement operation of the slider 13c, or the setting change of the displaying number, or the like. In this case, the display control unit 35a controls the display unit 13 so as to change the inter-image distance depending on the number of latent images between the images subsequent to the fifteenth image to be displayed in a list (in-vivo image P15), similarly to the images of the in-vivo images P1 to P15 described above. The display unit 13 changes each inter-image distance subsequent to such in-vivo image P15 depending on the number of latent images for every image under the control of such display control unit 35a.

As described above, in the third example , the plurality of in-vivo images are displayed in a list with each inter-image distance of the plurality of in-vivo images to be displayed in a list changed depending on the number of latent images between the images, and other configurations are similar to the second example. Thus, substantially similarly to the second example described above, an image display device, an image display method, and an image display program enabling the information of the latent image that latently exists between the images of the plurality of in-vivo images to be checked while observing the plurality of in-vivo images displayed in a list on the screen can be realized.

The magnitude relationship of the number of in-vivo images (i.e., number of latent images) that latently exist between the images of the plurality of in-vivo images can be easily compared while observing the plurality of in-vivo images extracted based on the desired image extracting condition from the in-vivo image group of the subject by using the image display device according to the third example. Thus, each image interval of the plurality of in-vivo images displayed in a list can be intuitively recognized, and thereby the positional relationship between the in-vivo images in the plurality of in-vivo images can be easily grasped. As a result, the in-vivo image can be efficiently observed in a short period of time while easily determining each capturing site of the plurality of in-vivo images.

In the first example described above, a different mark is added for every type of characteristic image that latently exists between the images of the plurality of in-vivo images to be displayed in a list to display the type of characteristic image that latently exists between such images, but numerical value information indicating the latent number of the characteristic image may be added to the mark of such characteristic image. In this case, the display control unit 15a counts the latent number of characteristic images between the images of the plurality of in-vivo images. The display control unit 15a adds the numerical value information indicating the latent image of the characteristic image between the images to the mark (e.g., marks M1 to M3) of the characteristic image described above, and controls the display unit 13 so as to add the mark of the characteristic image containing the numerical value information to a position between the images. The display unit 13 displays the mark of the characteristic image containing such numerical value information at a position between the images where the characteristic image latently exists under the control of such display control unit 15a. The user can recognize the type and the latent number of the characteristic image that latently exists between the images by visually recognizing the mark of the characteristic image containing such numerical value information, and can consequently more easily known the relevance and the positional relationship of the characteristic image that latently exists between such images and the in-vivo images to be displayed in a list.

In the first example described above, a single characteristic image corresponding to the mark is displayed in the image display window additionally displayed when the mark of the characteristic image is selected by the operation of the input unit 12 by the user, but if a plurality of characteristic images corresponding to the mark latently exist, the plurality of characteristic images corresponding to the mark may be displayed in the image display window.

Furthermore, in the second example described above, the numerical value information indicating the number of latent images between the images is added as the additional information indicating the information of the latent image that latently exists between the images of a plurality of in-vivo images to be displayed in a list, but is not limited thereto, and the additional information indicating the information of such latent image may be color information that differs depending on the number of latent images between the images. In this case, the display control unit 25a converts the number of latent images between the images of a plurality of in-vivo images to be displayed in a list to color information, adds the obtained color information as the additional information indicating the number of latent images between the images, and controls the display unit 13 so as to display the plurality of in-vivo images in a list. Such information changes from a warm color such as red to a cold color such a blue or from a cold color to a warm color with increase and decrease of the number of latent images between the images. The display unit 13 displays the plurality of in-vivo images in a list and displays the color information indicating the number of latent images between the images at a position between the images of the plurality of in-vivo images under the control of such display control unit 25a.

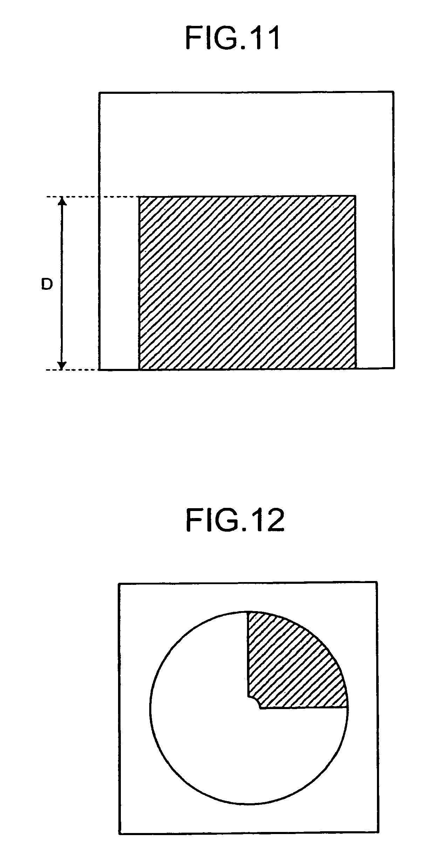

The additional information indicating the information of such latent image may be graph information that changes depending on the number of latent images between the images. FIG. 11 is a schematic view illustrating one specific example of the additional information indicating the information of the latent image. The display control unit 25a may add the graph information of bar graph-form, as illustrated in FIG. 11, to the display unit 13 as the additional information indicating the number of latent images between the images. In this case, the display control unit 25a increases and decreases the bar length D of the graph information illustrated in FIG. 11 depending on the increase and decrease of the number of latent images between the images, and controls the display unit 13 so as to display the plurality of in-vivo images in a list with the graph information having the bar length D corresponding to the number of latent images between such images added between the images. The display unit 13 displays the plurality of in-vivo images in a list and displays the graph information having such bar length D between the images of the plurality of in-vivo images under the control of such display control unit 25a.

The additional information indicating the information of such latent image may be color information that differs depending on the number of latent images between the images. FIG. 12 is a schematic view illustrating another specific example of the additional information indicating the information of the latent image. The display control unit 25a may add the graph information of circular graph-form, as illustrated in FIG. 12, to the display unit 13 as the additional information indicating the number of latent images between the images. In this case, the display control unit 25a increases and decreases the circular graph region (shaded portion illustrated in FIG. 12) of such graph information depending on the increase and decrease of the number of latent images between the images, and controls the display unit 13 so as to display the plurality of in-vivo images in a list with the graph information having the circular graph region corresponding to the number of latent images between such images added between the images. The display unit 13 displays the plurality of in-vivo images in a list and displays the graph information having such circular graph region between the images of the plurality of in-vivo images under the control of such display control unit 25a.

In the second embodiment described above, the additional information indicating the number of latent images between the images is added to a position between the images of the plurality of in-vivo images to be displayed in a list, but is not limited thereto, and the additional information indicating a captured time of the latent image that latently exists between the images of the plurality of in-vivo images may be added. In this case, the display control unit 25a calculates a total captured time of the latent image between the images of the plurality of in-vivo images based on the time data of each latent image (data indicating the captured time of the in-vivo image by the capsule endoscope 2) that latently exists between the images of the plurality of in-vivo images to be displayed in a list. The display control unit 25a executes a calculation process of the total captured time of the latent image between such images instead of executing the counting process of the number of latent images between the images, and controls the display unit 13 so as to display the plurality of in-vivo images in a list with the additional information indicating the total captured time of the latent image between such images added thereto. The display unit 13 displays the plurality of in-vivo images in a list and displays the additional information indicating the total captured time of the latent image between the images between the images of the plurality of in-vivo images under the control of such display control unit 25a.

Such additional information may be numerical value information indicating the total captured time of the latent image between the images, or may be color information that differs depending on the total captured time of the latent image between the images. If such additional information is the color information, the display control unit 25a converts the total captured time of the latent image between the images of a plurality of in-vivo images to be displayed in a list to color information. The display control unit 25a adds the obtained color information as the additional information indicating the total captured time of the latent image between the images, and controls the display unit 13 so as to display the plurality of in-vivo images in a list. Such color information changes, for example, from a warm color such as red to a cold color such a blue or from a cold color to a warm color with the increase and decrease of the total captured time of the latent image between the images.

Such additional information may be the graph information (see FIG. 11) of bar graph-form which the bar length D increases and decreases according to the increase and the decrease of the total captured time of the latent image between the images, or may be the graph information (see FIG. 12) which the circular graph region increases and decreases according to the increase and the decrease of the total captured time of the latent image between the images. In this case, the display control unit 25a increases and decreases the bar length D or the circular graph region of such graph information according to increase and decrease of the total captured time of the latent image between the images. The display control unit 25a controls the display unit 13 so as to display the plurality of in-vivo images in a list with the graph information having the bar length D or the circular graph region corresponding to the total captured time of the latent image between such images added between the images.

In the second example described above, additional information indicating the number of latent images between the images is added to a position between the images of the plurality of in-vivo images to be displayed in a list, but is not limited thereto, and the color information may be added to each image frame of the plurality of in-vivo images as the additional information indicating the information of the latent image that latently exists between the images of the plurality of in-vivo images.

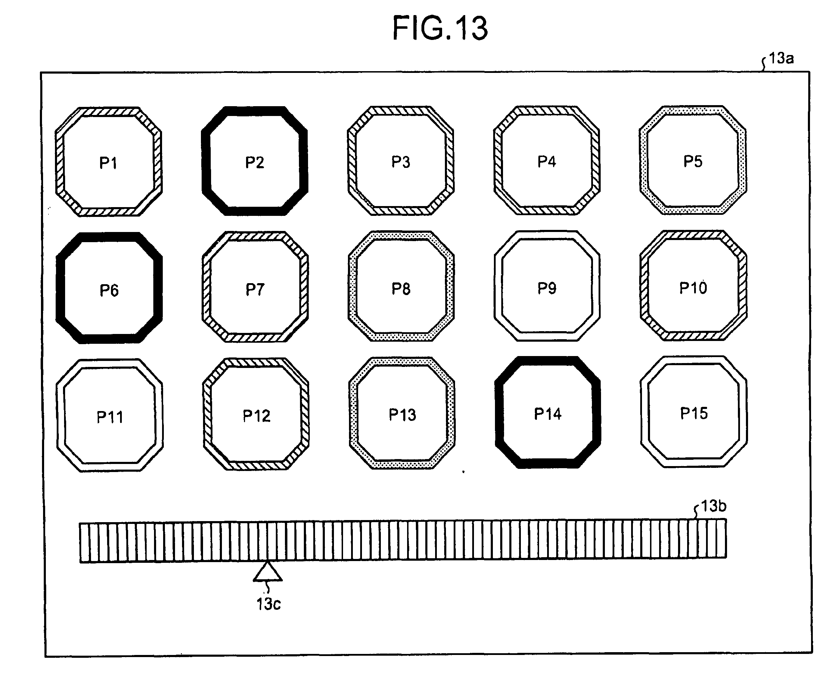

FIG. 13 is a schematic view illustrating a modified example of a list-display mode of the in-vivo images by the image display device according to the second example. Specifically, the display control unit 25a converts the number of latent images or the total captured time of the latent image between the images of a plurality of in-vivo images to be displayed in a list to color information. The display control unit 25a adds the obtained color information to each image frame as the additional information indicating the number of latent images or the total captured time of the latent image between the images, and controls the display unit 13 so as to display the plurality of in-vivo images in a list. Such color information changes, for example, from a warm color such as red to a cold color such a blue or from a cold color to a warm color with the increase and decrease of the number of latent images or the total captured time of the latent image between the images. The display unit 13 displays the plurality of images in a list, and displays the color information indicating the number of latent images or the total captured time of the latent image between such images at each image frame of the plurality of in-vivo images under the control of such display control unit 25a, as illustrated in FIG. 13.

In the third example described above, each inter-image distance of the plurality of in-vivo images to be displayed in a list is changed depending on the number of latent images between the images, but is not limited thereto, and each inter-image distance of the plurality of in-vivo images may be changed depending on the captured time of the latent image between the images. In this case, the display control unit 35a calculates the total captured time of the latent image between the images of the plurality of in-vivo images based on the time data of each latent image (data indicating the captured time of the in-vivo image by the capsule endoscope 2) that latently exists between the images of the plurality of in-vivo images to be displayed in a list. The display control unit 35a executes a calculation process of the total captured time of the latent image between such images instead of executing the counting process of the number of latent images between the images, and converts the total captured time of the latent image between such images to an inter-image distance. The display control unit 35a controls the display unit 13 so as to display in a list the plurality of in-vivo images spaced apart by the obtained inter-image distance. The inter-image distance controlled by such display control unit 35a increases with increase in the total captured time of the latent image between the images, and decreases with decrease in the total captured time of the latent image between the images. The display unit 13 displays in a list the plurality of images to be displayed in a list in a mode in which each inter-image distance is changed depending on the total captured time of the latent image between the images under the control of such display control unit 35a.

In the first to the third examples described above, the mark indicating the information of the characteristic image or the additional information indicating the number of latent images is added between the images of the plurality of in-vivo images or each inter-image distance is changed depending on the number of latent images between the images when displaying the plurality of in-vivo images in a list, but is not limited thereto, and an image display device appropriately combining the first to the third examples may be obtained.

For instance, the image display device 4 according to the first embodiment may display the mark of the characteristic image between the images, as described above, and display the additional information indicating the number of latent images or the total captured time to a position between the images of the plurality of in-vivo images to be displayed in a list, similar to the image display device 24 according to the second example. In this case, the display control unit 15a of the image display device 4 may have a function similar to the display control unit 25a according to the second example. The image display device 4 according to the first example may display the mark of the characteristic image between the images, as described above, and display in a list a plurality of in-vivo images to be displayed in a list while changing each inter-image distance depending on the number of latent images or the total captured time between the images, similarly to the image display device 34 according to the third example. In this case, the display control unit 15a of the image display device 4 may have a function similar to the display control unit 35a according to the third example.

In the first to the third examples described above, a threshold value related to the similarity of the in-vivo images is set as the image extracting condition for extracting the in-vivo images to be displayed in a list, but such image extracting condition is not limited to the threshold value related to the similarity of the in-vivo images. For instance, such image extracting condition may be a threshold value related to a predetermined color level such as red or green in the in-vivo image, a threshold value related to a level of an average color of the in-vivo image, a threshold value related to a spreading level (the number of pixels of a predetermined color) of a predetermined color in the in-vivo image, a frame interval for decimating or extracting the in-vivo images from the in-vivo image group captured in time-series, or may be a combination of at least two of the above.

As described above, in the image display device according to the first, the second, or the third examples, an image acquiring unit extracts a series of images captured in time-series, the image extracting unit extracts a plurality of images from the above-described series of images, the display unit displays the plurality of images extracted by the above-described image extracting unit in a list, and the display control unit controls the above-described display unit so as to display the plurality of images in a list in a display mode of illustrating the information of the latent image that latently exists between the images of the plurality of images. Thus, an image display device capable of checking the information of the latent image that latently exists between the images of the plurality of in-vivo images while observing the plurality of in-vivo images displayed in a list on the screen can be realized.

In the image display method according to the first, the second, or the third examples, an image extracting step of extracting a plurality of images from a series of images captured in time-series is performed, and a list-display step of displaying the plurality of images in a list in a display mode of illustrating the information of the latent image that latently exists between the images of the plurality of images extracted in the above-described image extracting step is performed. Thus, an image display method capable of checking the information of the latent image that latently exists between the images of the plurality of in-vivo images while observing the plurality of in-vivo images displayed in a list on the screen can be realized.

Furthermore, in the image display program according to the first, the second, or the third examples, an image extracting procedure of extracting a plurality of images from a series of images captured in time-series is performed, and a list-display procedure of displaying the plurality of images in a list in a display mode of illustrating the information of the latent image that latently exists between the images of the plurality of images extracted in the above-described image extracting procedure is performed. Thus, an image display program capable of checking the information of the latent image that latently exists between the images of the plurality of in-vivo images while observing the plurality of in-vivo images displayed in a list on the screen can be realized.

### Fourth Example

FIG. 14 is a block diagram schematically illustrating one configuration example of an image display device according to a fourth example.

As illustrated in FIG. 14, an image display device 44 according to the fourth example includes a data input/output unit 42 for inputting and outputting image data, the display unit 13 for displaying image data and GUI (Graphical User Interface), etc., the input unit 12 for inputting various types of information, the storage unit 14 for storing the image data, etc., input by the data input/output unit 42, a control unit 45 for controlling each units of such image display device 44. An in-vivo information acquiring system according to the fourth embodiment includes the image display device 44 in place of the image display device 4 of the in-vivo information acquiring system (see FIG. 1) according to the first example described above. Other configurations are the same as any one of the first to the third examples, and the same reference numerals are denoted for the same components.

The data input/output unit 42 functions as an image acquiring unit for acquiring the image group captured in time-series. Specifically, the data input/output unit 42 is constructed using a drive having a structure to which a portable recording medium such as a card memory is attachable. The data input/output unit 42 reads the saved data in the attached recording medium under the control of the control unit 45 and inputs the acquired saved data to the control unit 45. The data input/output unit 42 writes data write-instructed by the control unit 45 to the attached recording medium. The data acquired from the recording medium by the data input/output unit 42 includes the group of in-vivo images sequentially captured in time-series by the capsule endoscope, the time data indicating the captured time of each image in the image group, the patient data such as patient name and patient ID specifying the subject to whose organs the capsule endoscope is inserted, and the like. The data written to the recording medium by the data input/output unit 42 includes the patient information such as patient name and patient ID specifying the subject.

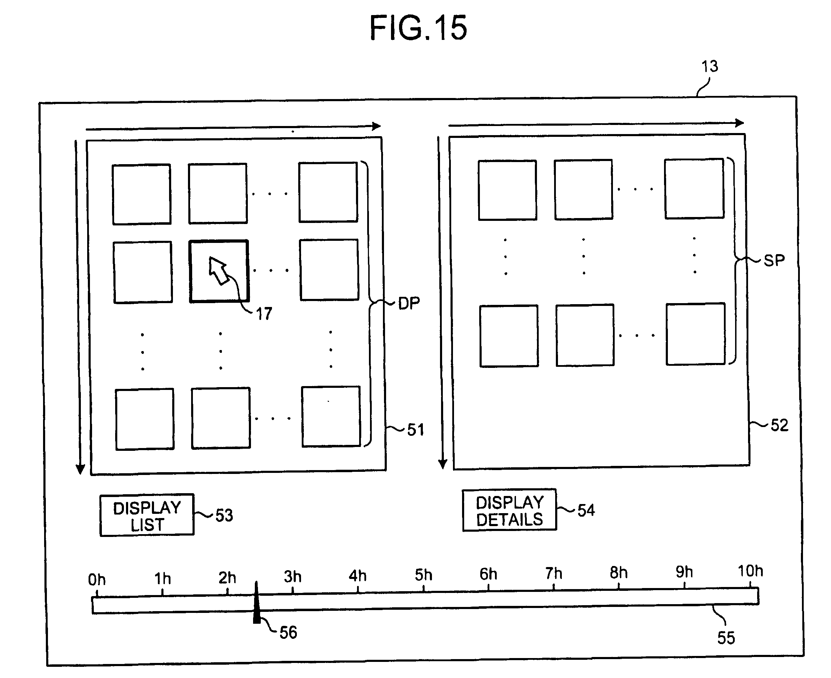

The display unit 13 functions as a display unit for displaying the image group acquired by the data input/output unit 42. Specifically, the display unit 13 is constructed using a display device such as a CRT display and a liquid crystal display, and displays various types of information such as images display instructed by the control unit 45 and GUI. FIG. 15 is a schematic view illustrating one specific example of the display mode of the display unit in the fourth example. As illustrated in FIG. 15, the display unit 13 is formed with a list-display area 51 and a detailed-display area 52. The display unit 13 displays a representative image group DP display instructed by the control unit 45 in a list-display area 41 in a still image state, and displays a latent image group SP display instructed by the control unit 45 in the detailed-display area 52 in a still image state. In this case, the display unit 13 displays the representative image group DP in a list in a mode in which each image is arrayed in the time-series order as illustrated with an arrow in FIG. 15, and displays the latent image group SP in a list in the same array mode as such representative image group DP.

The representative image in such representative image group DP is an image extracted based on a predetermined extracting condition from the image group acquired by the data input/output unit 42. The latent image in the latent image group SP is the remaining image excluding the representative image of the image group, and is the image that latently exists between the representative images in the representative image group DP displayed in a list.

The display unit 13 displays the a "DISPLAY LIST" icon 53 which is the GUI for inputting the instructing information for instructing the list-display of the representative image group DP, a "DISPLAY DETAILS" icon 54 which is the GUI for inputting the instructing information for instructing the list-display of the latent image group SP, a graphic bar 55 and an indicator 56 which are the GUI for illustrating the time position of the image selected from the representative image group DP and the latent image group SP currently being displayed, and the mouse cursor 17 that is movable in response to the operation of the input unit 12.

The graphic bar 55 has a time scale as illustrated in FIG. 15, and illustrates the overall time position of the series of images captured by the capsule endoscope. The graphic bar 55 is created based on the color information of the series of images captured by the capsule endoscope, and thereby, illustrates by its color distinction of the in-vivo and in-vitro images, distinction of organs, presence of residual dross, and the like. The time scale of such graphic bar 55 illustrates, for example, the elapsed time from the captured time of the image of the first frame captured by the capsule endoscope. The indicator 56 moves along such graphic bar 55, and illustrates the time position of either the image of the representative image group DP or the latent image group SP currently being displayed.

The display unit 13 highlights the image selected from such representative image group DP and the latent image group SP. In FIG. 15, the representative image, on which the mouse cursor 17 is placed, of the representative image group DP is highlighted. The display unit 13 highlights the image corresponding to the time position pointed by the indicator 56 on the graphic bar 55, that is, the representative image in the list-display area 51 or the latent image in the detailed-display area 52. Although not particularly illustrated in FIG. 15, the display unit 13 displays the various types of information useful in observing the in-vivo image of the subject such as the patient data of the subject to whose organs the capsule endoscope is inserted, that is, the subject corresponding to the representative image group DP and the latent image group SP.

The input unit 12 is constructed using the input device such as keyboard and mouse, and inputs various types of information to the control unit 45 in response to the input operation by the user. Specifically, the input unit 12 inputs image specifying information for specifying one of the images being currently displayed on the display unit 13, various types of information for instructing to the control unit 45, and the like to the control unit 45. The image specifying information input by such input unit 12 includes representative image specifying information for designating a representative image contained in the representative image group DP of the list-display area 51 and latent image specifying information for designating a latent image contained in the latent image group SP of the detailed-display area 52. The instructing information input by such input unit 12 includes display instructing information for instructing the list-display of the representative image group DP, display instructing information for instructing the list-display of the latent image group SP, and displaying number instructing information for instructing the number of frames of the representative image group DP to be displayed in a list in the list-display area 51.

The input unit 12 inputs the representative image specifying information for designating the representative image by placing the mouse cursor 17 on the to-be-designated representative image of the representative image group DP of the list-display area 51 and clicking the same, and the input unit 12 inputs the latent image specifying information for designating the latent image by placing the mouse cursor 17 on the to-be-designated latent image of the latent image group SP of the detailed-display area 52 and clicking the same. The input unit 12 inputs the display instructing information for instructing the list-display of the representative image group DP by placing the mouse cursor 17 on the "DISPLAY LIST" icon 53 and clicking the same, and the input unit 12 inputs the display instructing information for instructing the list-display of the latent image group SP by placing the mouse cursor 17 on the "DISPLAY DETAILS" icon 54 and clicking the same.

The storage unit 14 is constructed using a large capacity recording medium such as RAM, EEPROM, or hard disc, and saves various types of data, etc., write-instructed by the control unit 45 and transmits the saved data read-instructed by the control unit 45 to the control unit 45. Specifically, the storage unit 14 includes an all-image folder 44a, which is a storage area, for storing the image group taken in by the above-described data input/output unit 42, and a representative image folder 44b, which is a storage area, for storing the representative image group DP displayed in a list in the list-display area 51 of the above-described display unit 13. Such storage unit 14 stores the image group captured in time-series by the capsule endoscope, that is, the series of images including the in-vivo images of the subject in the all-image folder 44a, and stores the representative image group DP extracted from the image group in the representative image folder 44b. In addition to such image data, the storage unit 14 stores the time data of each image in the image group captured by the capsule endoscope, the similarity of each image between the adjacent images of the image group, and the patient data of the subject, and the like. In this case, the time data and the similarity for every image are associated with each image data saved in the storage unit 14.

The control unit 45 controls each units of the image display device 44, and is constructed using a CPU for executing a processing program, a ROM recorded with the processing program, etc., in advance, and a RAM for temporarily storing calculation parameter, etc., of each process. Specifically, the control unit 45 controls each operation of the above-described data input/output unit 42, the display unit 13, the input unit 12, and the storage unit 14, and controls the input and the output of signals between each configuring units. More specifically, the control unit 45 controls the data input/output unit 42 in accordance with the instructing information input by the input unit 12 to acquire the saved data of the recording medium in the data input/output unit 42. The control unit 45 controls the storage unit 14 so as to store the acquired saved data, that is, the image group by the capsule endoscope, the time data of each image, and the like. The control unit 45 controls the display unit 13 so as to highlight the representative image designated by the representative image specifying information input by the input unit 12, and move the indicator 56 to the time position of the graphic bar 55 corresponding to the representative image. Similarly, the control unit 45 controls the display unit 13 so as to highlight the latent image designated by the latent image specifying information input by the input unit 12, and move the indicator 56 to the time position of the graphic bar 55 corresponding to the latent image.

The control unit 45 includes an image extracting unit 45a for extracting the representative images displayed in a list in the list-display area 51 of the display unit 13, and an image selecting unit 45b for selecting the latent images displayed in a list on the detailed-display area 52 of the display unit 13. The image extracting unit 45a extracts a plurality of representative images from a series of images saved in the all-image folder 44a of the storage unit 14. Specifically, the control unit 45 sets the extracting number of the representative images in accordance with the displaying number instructing information input by the input unit 12, that is, the displaying number of representative images to be displayed in a list in the list-display area 51 of the display unit 13. The image extracting unit 45a calculates the similarity between the adjacent images in the image group for every image based on the color information of each image in the image group acquired by the data input/output unit 42 or the movement vector, etc., between the adjacent images. Such image extracting unit 45a extracts the representative images for the number of frames, the number being same as the set displaying number, from the image group saved in the all-image folder 44a based on the similarity of each image. In this case, the image extracting unit 45a extracts the representative images for the set displaying number in the ascending order or the descending order of the similarity of each image. The control unit 45 controls the storage unit 14 so as to save the data of a plurality of representative images extracted by such image extracting unit 45a in the representative image folder 44b. The control unit 45 controls the display unit 13 so as to display such plurality of representative images in a list in the list-display area 51 in accordance with the display instructing information corresponding to the "DISPLAY LIST" icon 53. The plurality of representative images extracted by such image extracting unit 45a are displayed, for example, in a list in the list-display area 51 of the display unit 13 as the representative image group DP illustrated in FIG. 15.

The image selecting unit 45b selects the image, that is, the latent image latently existing between the representative images of the representative image group DP displayed in a list in the list-display area 51 of the display unit 13 by one or more frames from the image group saved in the all-image folder 44a. Specifically, the image selecting unit 45b selects the latent image of one or more frames represented by the representative image of one or more frames designated by the above-described representative image specifying information, that is, the latent image of one or more frames latently existing between the representative image designated by the representative image specifying information and another representative image adjacent to at least the earlier side or the later side of the representative image from the image group saved in the all-image folder 44a. The control unit 45 controls the display unit 13 so as to display the latent images of one or more frames selected by such image selecting unit 45b in a list in the detailed-display area 52 in accordance with the display instructing information corresponding to the "DISPLAY DETAILS" icon 54. The latent images of one or more frames extracted by such image selecting unit 45b are displayed, for example, in a list in the detailed-display area 52 of the display unit 13 as the latent image group SP illustrated in FIG. 15.

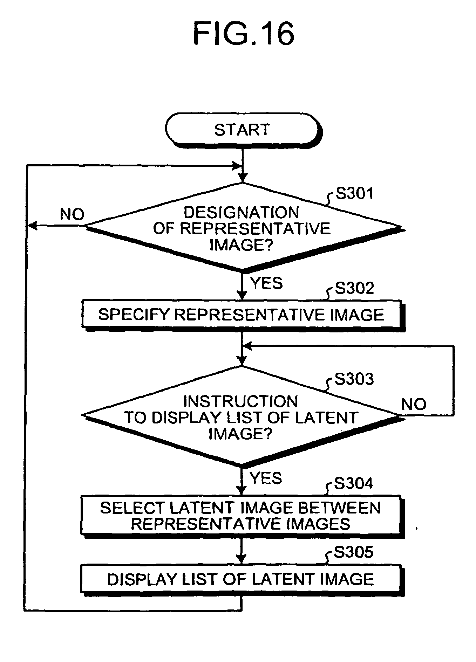

The operation of the image display device 44 according to the fourth example will be described below illustrating a case of displaying the representative image group DP in a list in the list-display area 51 of the display unit 13, and displaying the latent image latently existing between the representative images of the representative image group DP in a list in the detailed-display area 52 of the display unit 13 by way of example. FIG. 16 is a flowchart illustrating a processing procedure of the control unit of the image display device until displaying the latent image latently existing between the representative images in a list. The image display device 44 displays in a list the latent image group SP represented by the representative image designated from the representative image group DP displayed in a list in the list-display area 51 in the detailed-display area 52.

In other words, as illustrated in FIG. 16, the control unit 45 of the image display device 44 determines the presence of designation of one of the representative images contained in the representative image group DP in the list-display area 51 of the display unit 13 (step S301). In step S301, the control unit 45 determines that the designation of the representative image corresponding to the representative image specifying information is made (step S301, Yes) if the representative image specifying information is input by the input unit 12, and specifies the representative image designated by the input representative image specifying information from the representative image group DP (step S302).

The control unit 45 then determines the presence of the instruction to display in a list the latent image represented by the representative image specified in step S302 (step S303). In step S303, the control unit 45 determines that the instruction to display the latent image in a list is made (step S303, Yes) if the display instructing information corresponding to the "DISPLAY DETAIL" icon 54 is input by the input unit 12, and selects the latent image between the representative images including the representative image specified in step S302 (step S304).

In step S304, the image selecting unit 45b searches for the representative image specified in step S302 and another representative image adjacent in time-series from each representative image saved in the representative image folder 44b of the storage unit 14 based on the time information associated with each representative image. The image selecting unit 45b then selects the latent image of one or more frames latently existing between the another representative image adjacent to the specified representative image and the specified representative image from the image group saved in the all-image folder 44a of the storage unit 14.

Subsequently, the control unit 45 controls the display unit 13 so as to display in a list the latent images of one or more frames selected in step S304 in the detailed-display area 52 (step S305), and then returns to step S301 and repeats the processing procedures step S301 and subsequent steps. In step S305, the display unit 13 displays, for example, in a list the latent image group SP in the detailed-display area 52 as illustrated in FIG. 15 under the control of such control unit 45.

The control unit 45 determines that the designation of the representative image is not made (step S301, No) if the representative image specifying information is not input in step S301, and repeats step S301. The control unit 45 determines that the instruction to display in a list the latent images (step S303, No) is not made if the display instructing information corresponding to the "DISPLAY DETAILS" icon 54 is not input in step S303, and repeats step S303.

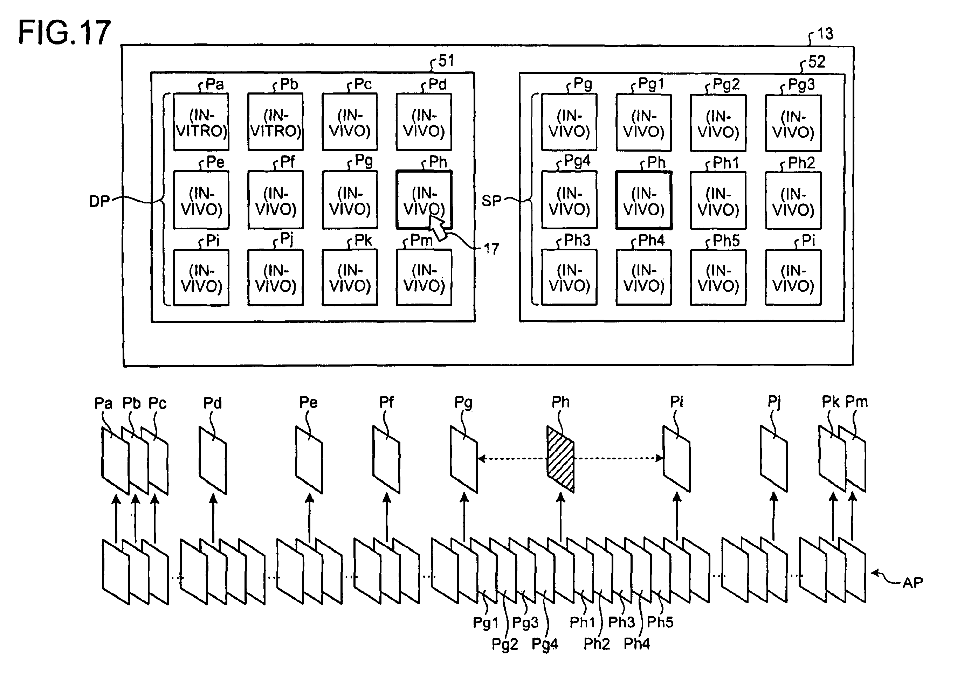

With the case in which the control unit 45 sets the display number of representative images as 12 frames based on the input information of the input unit 12 by way of example, the operation of the image display device 44 in displaying in a list the latent image latently existing between the representative images of the representative image group DP of the 12 frames displayed in a list in the list-display area 51 of the display unit 13 in the detailed-display area 52 of the display unit 13 will be specifically described. FIG. 17 is a schematic view specifically describing the operation of the image display device according to the fourth example of the present invention. The operation of the image display device 44 will be specifically described with reference to FIGS. 14, 2 and FIG. 17.

The control unit 45 of the image display device 44 according to the fourth embodiment acquires the image group AP captured in time-series order by the capsule endoscope and the time data of each image, etc., from the data input/output unit 42. The control unit 45 calculates the similarity between the adjacent images in the acquired image group AP for every image, and associates the obtained similarity and each time data between the adjacent images and each image of the image group AP. The control unit 45 saves the image group AP with which such similarity and the time data are associated for every image in the all-image folder 44a of the storage unit 14. The control unit 45 sets the number of frames of the representative images displayed in a list in the list-display area 51 of the display unit 13 to, for example, 12 frames in accordance with the display number instructing information input by the input unit 12.

When the display instructing information corresponding to the "DISPLAY LIST" icon 53 illustrated in FIG. 15 is input, the control unit 45 controls the display unit 13 so as to display the representative image group DP of 12 frames of the image group AP in the list-display area 51. In this case, the image extracting unit 45a extracts the representative images Pa, Pb, Pc, Pd, Pe, Pf, Pg, Ph, Pi, Pj, Pk, and Pm of 12 frames of the same number as the set displaying number in ascending order of the similarity from the image group AP as illustrated in FIG. 17. The control unit 45 saves the extracted representative images Pa, Pb, Pc, Pd, Pe, Pf, Pg, Ph, Pi, Pj, Pk, and Pm of 12 frames in the representative image folder 44b of the storage unit 14 in association with each similarity and each time data, and controls the display unit 13 so as to display the representative images Pa, Pb, Pc, Pd, Pe, Pf, Pg, Ph, Pi, Pj, Pk, and Pm of 12 frames in the list-display area 51 as the representative image group DP.

The representative images Pa, Pb, Pc, Pd, Pe, Pf, Pg, Ph, Pi, Pj, Pk, and Pm extracted in ascending order of the similarity are images of the top 12 frames from low similarity of the image group AP. Specifically, the representative images Pa, Pb are images captured by the capsule endoscope before being inserted into the subject, that is, the in-vitro images, and the remaining representative images Pc, Pd, Pe, Pf, Pg, Ph, Pi, Pj, Pk, Pm are images of the inside of the organ captured by the capsule endoscope inserted into the subject, that is, the in-vivo images.

As illustrated in FIG. 17, when the mouse cursor 17 is placed on the representative image Ph of the representative image group DP and click operated to input the representative image specifying information for designating the representative image Ph from the input unit 12 to the control unit 45, the control unit 45 controls the display unit 13 so as to highlight the representative image Ph designated by the input representative image specifying information. Thereafter, if the display instructing information corresponding to the "DISPLAY DETAILS" icon 54 illustrated in FIG. 15 is input by the input unit 12, the control unit 45 controls the display unit 13 so as to display the latent image group SP latently existing between the representative images including the representative image Ph in the detailed-display area 52.

Specifically, the image selecting unit 45b specifies the representative image Ph designated by the representative image specifying information and the representative images Pg, Pi adjacent to the representative image Ph in time-series from the representative images Pa, Pb, Pc, Pd, Pe, Pf, Pg, Ph, Pi, Pj, Pk, Pm of the representative image folder 44b. The image selecting unit 45b selects all latent images Pg1 to Pg4 latently existing between such representative image Pg and the representative image Ph, and all latent images Ph1 to Ph5 latently existing between the representative image Ph and the representative image Pi from the image group PA in the all-image folder 44a. The control unit 45 controls the display unit 13 so as to display the latent images Pg1 to Pg4, and Ph1 to Ph5 selected by such image selecting unit 45b in the detailed-display area 52 as the latent image group SP. In this case, the control unit 45 may display the image group in which the latent images Pg1 to Pg4, Ph1 to Ph5 selected by such image selecting unit 45b include the representative images Pg, Ph, Pi representing such latent images as the latent image group SP, as illustrated in FIG. 17.

Such latent images Pg1 to Pg4 are in-vivo images represented by the representative image Pg, and exist in time-series order between the representative image Pg and the representative image Ph. The latent images Ph1 to Ph5 are in-vivo images represented by the representative image Ph, and exist in time-series order between the representative image Ph and the representative image Pi.

When the representative image specifying information for designating the representative image other than the representative image Ph and the display instructing information corresponding to the "DISPLAY DETAILS" icon 54 are input as well, such control unit 45 controls the display unit 13 so as to display the latent image group latently existing between the representative images including the representative image designated by the input representative image specifying information in the detailed-display area 52, similarly to the case of the representative image Ph.

As described above, in the fourth example, a plurality of representative images are extracted from the image group captured in time-series, the extracted plurality of representative images are displayed in a list in the display unit, and one or more latent images latently existing between the representative images including the representative image designated from the plurality of representative images displayed in a list are selected from the image group and the one or more selected latent images are displayed in a list in the display unit. Thus, the plurality of representative images of relatively low similarity can be displayed in a list from the image group captured in time-series, and the latent image latently existing between the desired representative images in the plurality of representative images displayed in a list can be easily displayed in a list, for example, in a still image state. Thus, even if the remaining images other than the plurality of representative images displayed in a list, that is, the image of a characteristic site to be observed such as the lesion part or the bleeding part of the inside of the subject is included in the latent image, the possibility which the image of the characteristic site is screen displayed increases by the list-display of such latent images, and consequently, an image display device capable of suppressing the overlook of the image of the characteristic site in the inside of the subject and enabling the in-vivo image group of the subject to be observed in a short period of time can be realized.

### First Embodiment

A first embodiment of the present invention will now be described. In the fourth example described above, the representative image group extracted from the image group captured in time-series and the latent image of one or more frames latently existing between the representative images in such representative image group are displayed in a list in each display area of the display unit 13, but in the first embodiment, the representative image designated from the representative image group displayed in a list on the display unit 13 and the latent image represented by such representative image may be set to non-display.

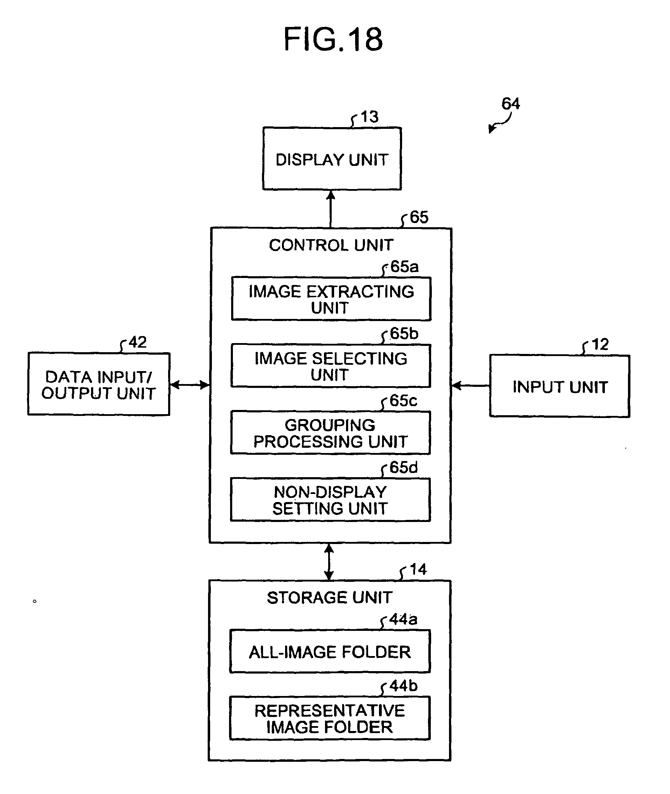

FIG. 18 is a block diagram schematically illustrating one configuration example of an image display device according to the first embodiment of the present invention.

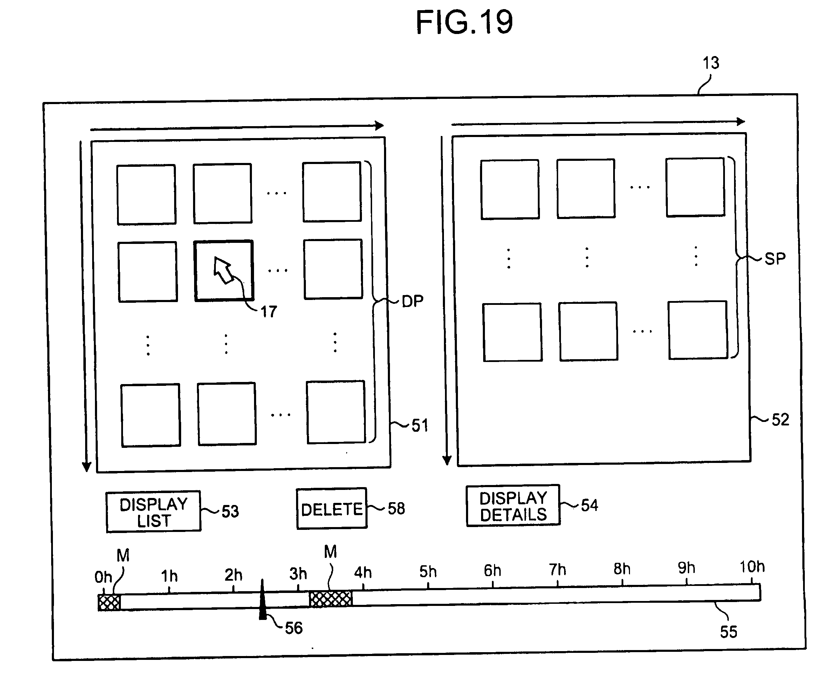

FIG. 19 is a schematic view illustrating one specific example of a display mode of the display unit according to the first embodiment of the present invention. As illustrated in FIG. 18, an image display device 64 according to the first embodiment includes a control unit 65 in place of the control unit 45 of the image display device 44 according to the fourth example described above. In the image display device 64, the display unit 13 displays a "DELETE" icon 58, which is a GUI, for instructing the non-display setting of the image, and displays a mark M indicating the time position of the non-display set image on the graphic bar 55, as illustrated in FIG. 19. An in-vivo information acquiring system according to the first embodiment includes the image display device 64 in place of the image display device 4 of the in-vivo information acquiring system (see FIG. 1) according to the first example described above. Other configurations are the same as any one of the first to the fourth examples, and same reference numerals are denoted for the same components.

The control unit 65 includes an image extracting unit 65a in place of the image extracting unit 45a of the control unit 45 according to the fourth example described above, and also includes an image selecting unit 65b in place of the image selecting unit 45b. The control unit 65 also includes a grouping processing unit 65c for performing a grouping process of the images, and a non-display setting unit 65d for performing a non-display setting process of the image. The control unit 65 controls the display unit 13 so as to display the mark M as illustrated in FIG. 19 at the time position of the graphic bar 55 corresponding to the image set to be a non-display target by the non-display setting unit 65d of the image group saved in the all-image folder 44a of the storage unit 14. The control unit 65 has a function similar to the control unit 45 according to the fourth example described above other than each function related to the image extracting unit 65a, the image selecting unit 65b, the grouping processing unit 65c, or the non-display setting unit 65d and the display control function of the mark M.

The image extracting unit 65a re-extracts the representative images of the same number as the representative image deleted from the display target of the representative image group DP displayed in a list in the list-display area 51 of the display unit 13 as illustrated in FIG. 19, that is, the non-display set representative image from the image group in the all-image folder 44a. In this case, the image extracting unit 65a extracts, as a new representative image, the latent image having similarity of the order following the already extracted representative image from the latent image, which is not non-display set, of the image group of the all-image folder 44a by the same number as the non-display set representative image. The control unit 65 controls the display unit 13 so as to display in a list the new representative image re-extracted by such image extracting unit 65a in the list-display area 51 in addition to the already displayed representative image. In this case, the control unit 65 controls the display unit 13 so as to display in a list the representative image group DP including such new representative image arranged in time-series order. The image extracting unit 65a has a function similar to the image extracting unit 45a of the control unit 45 according to the fourth example described above other than the re-extracting function of the representative image.

The image selecting unit 65b selects the latent image, which is not non-display set by the non-display setting unit 65d, from the latent images latently existing in the image group AP. In this case, the image selecting unit 65b selects the latent image from the image group of the all-image folder 44a. The latent image selected by such image selecting unit 65b is displayed in a list in the detailed-display area 52 of the display unit 13 as illustrated with the latent image group SP illustrated in FIG. 19, for example, under the control of the control unit 65.

When the non-display instructing information for instructing the non-display setting of the image is input by the input unit 12, the grouping processing unit 65c groups the representative image specified by the representative image specifying information described above, and the latent image of one or more frames latently existing between two representative images adjacent on the earlier side and the later side of the specified representative image. Such grouping processing unit 65c performs the grouping process with each image in time-series order including the representative image specified from the representative image group DP displayed in a list in the list-display area 51 and all the latent images represented by such representative image as illustrated in FIG. 19, for example, as a series of image groups. The non-display instructing information is input from the input unit 12 to the control unit 65, for example, when the click operation of the input unit 12 is performed with the mouse cursor 17 placed on the "DELETE" icon 58 illustrated in FIG. 19.

The non-display setting unit 65d sets the image to be deleted from the display target of the display unit 13 as the non-display image of the image group in the all-image folder 44a. Specifically, when the non-display instructing information is input by the input unit 12, the non-display setting unit 65d adds a non-display flag indicating to be the image of non-display target of the display unit 13 to the representative image specified by the representative image specifying information described above or each image in the series of image groups grouped by the grouping processing unit 65c described above. The non-display setting unit 65d thereby sets the specified representative image or each image in the series of image groups to the non-display image. The non-display setting unit 65d repeats the non-display setting process of such image every time the non-display instructing information described above is input. The control unit 65 controls the display unit 13 so as not to display the representative image and the latent image added with such non-display flag.

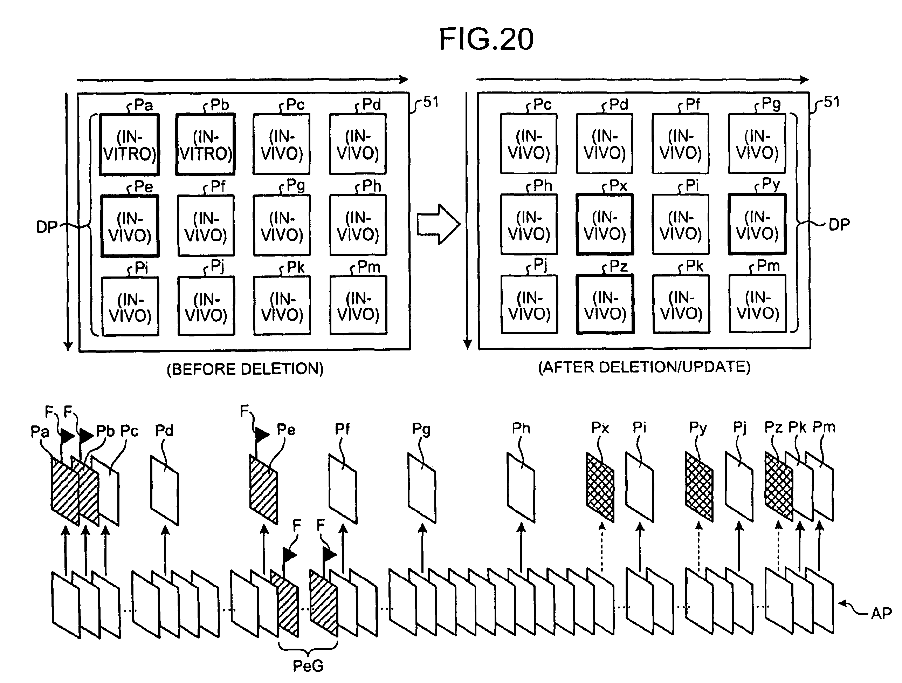

The operation of the image display device 64 according to the first embodiment of the present invention will be specifically described by illustrating a case of displaying the representative image group DP of 12 frames in a list in the list-display area 51 of the display unit 13. FIG. 20 is a schematic view specifically describing the operation of the image display device according to the first embodiment of the present invention. The operation of the image display device 64 according to the first embodiment is similar to the image display device 44 according to the fourth example described above excluding the operation of non-display setting the image and the operation of adding a new representative image of the same number as the non-display set representative image in the list-display. The operation of the image display device 64 when non-display setting the image and the operation of the image display device 64 when adding a new representative image of the same number as the non-display set representative image in the list-display will be specifically described below with reference to FIGS. 18 to FIG. 20.

When the representative image specifying information for designating the representative images Pa, Pb, Pe of the representative image group DP of 12 frames displayed in the list-display area 51 of the display unit 13 is sequentially input by the input unit 12, the control unit 65 of the image display device 64 according to the fifth embodiment controls the display unit 13 so as to highlight each representative image Pa, Pb, Pe designated by each representative image specifying information. The representative image specifying information of each representative image Pa, Pb, Pe is input by sequentially repeating the operation of the input unit 12 of clicking with the mouse cursor 17 placed on the representative image to be designated.

If the non-display instructing information is input by the input unit 12 in such state, the control unit 65 deletes at least the designated representative images Pa, Pb, Pe from the display target of the display unit 13, as described above. In this case, the non-display setting unit 65d adds the non-display flag F to the representative images Pa, Pb designated by the representative image specifying information of the representative images Pa, Pb, Pc, Pd, Pe, Pf, Pg, Ph, Pi, Pj, Pk, Pm extracted from the image group AP by the image extracting unit 65a. The non-display setting unit 65d thus sets such representative images Pa, Pb as the non-display images.

The grouping processing unit 65c performs the grouping process with each image in time-series order including the representative image Pe designated by the representative image specifying information and all latent images represented by the representative image Pe as a series of image groups PeG. All latent images contained in the image group PeG are, for example, latent images latently existing between the representative image Pf adjacent subsequent to the representative image Pe and the representative image Pe. The non-display setting unit 65d adds the non-display flag F to each image in the image group PeG grouped by such grouping processing unit 65c, that is, the representative image Pe and all latent images represented by the representative image Pe. The non-display setting unit 65d thereby sets each image of such image group PeG to the non-display image.

When the non-display flag F is added to the representative images Pa, Pb and each image in the image group PeG, the control unit 65 sets each image added with the non-display flag F of the image group AP in the all-image folder 44a of the storage unit 14 as the non-display target of the display unit 13, and sets the representative images Pa, Pb, Pe added with the non-display flag F of the representative image group DP in the representative image folder 44b of the storage unit 14 as the non-display targets of the display unit 13.

The image extracting unit 65a thereafter newly re-extracts the representative images of the same number as, that is, for three frames of the representative images Pa, Pb, Pe added with the non-display flag F from the image group AP. In this case, the image extracting unit 65a extracts the latent image having similarity of the order following the already extracted representative images Pc, Pd, Pf, Pg, Ph, Pi, Pj, Pk, Pm as the new representative images Px, Py, Pz from the images not added with the non-display flag F of the image group AP. The control unit 65 controls the storage unit 14 so as to save each data of the new representative images Px, Py, Pz re-extracted by such image extracting unit 65a in the representative image folder 44b in association with the above-described time data and the similarity.

Subsequently, the control unit 65 controls the display unit 13 so as to non-display the representative images Pa, Pb, Pe added with such non-display flag F and to add the representative images Px, Py, Pz of the same number as the non-display representative images Pa, Pb, Pe in the list-display of the list-display area 51 from the representative image group DP already displayed in a list in the list-display area 51. In this case, the control unit 65 controls the display unit 13 so as to display in a list the representative image group DP, in a mode in which the new representative images Px, Py, Pz area added to the existing representative images Pc, Pd, Pf, Pg, Ph, Pi, Pj, Pk, Pm, arranged in time-series order.

The display unit 13 deletes the representative images Pa, Pb, Pe of non-display target and additionally displays the new representative images Px, Py, Pz in the list-display area 51 so that the display mode changes from the state before deletion to the state after deletion/update illustrated in FIG. 20 under the control of such control unit 65. In this case, the display unit 13 additionally displays the representative image Px, which is adjacent to and is on the later side of the representative image Ph and is adjacent to and is on the earlier side of the representative image Pi in time-series, between the representative images Ph, Pi, additionally displays the representative image Py, which is adjacent to and is on the later side of the representative image Pi and is adjacent to and is on the earlier side of the representative image Pj in time-series, between the representative images Pi, Pj, and additionally displays the representative image Pz, which is adjacent to and is on the later side of the representative image Pj and is adjacent to and is on the earlier side of the representative image Pk in time-series, between the representative images Pj, Pk.

The control unit 65 excludes each latent image in the image group PeG added with the above-described non-display flag F from the display target to the detailed-display area 52 of the display unit 13. In other words, even if the representative image specifying information for designating the representative image Pd or the representative image Pf and the display instructing information corresponding to the "DISPLAY DETAILS" icon 54 are input, the control unit 65 controls the display unit 13 so as not to display each latent image in the image group PeG added with such non-display flag F in the detailed-display area 52.

The non-display representative images Pa, Pb, Pe added with the non-display flag F described above are images unnecessary for the observation of the inside of the organ of the subject. Specifically, the representative images Pa, Pb are images of the exterior of the subject, and the representative image Pe is the in-vivo image including great amount of residual dross such as stool and few amount of drawing of the inner wall of the organs. The latent image in the image group PeG represented by such representative image Pe is the in-vivo image including great amount of residual loss, similar to the representative image Pe, that is, the image unnecessary for the observation of the inside of the organ of the subject. The image display device 64 having the above-described configuration easily deletes the representative images Pa, Pb, Pe from one list-display in the list-display area 51 by designating the representative images Pa, Pb, Pe, which are images unnecessary for such observation, and additionally adds the new representative images Px, Py, Pz, which have the possibility of being the in-vivo images capturing a characteristic site such as the lesion part or the bleeding part of the inside of the subject, by the number of deleted images. As a result, the image display device 64 can reduce the images unnecessary for the observation from the list-display and enhance the possibility of displaying the images useful for the observation.

The images unnecessary for the observation of the inside of the organ of the subject include, in addition to the image of the exterior of the subject and the in-vivo image with great amount of residual loss, a normal in-vivo image in which the characteristic site such as the lesion part and the bleeding part is not captured, and an image of a subject successively captured with the capsule endoscope in the subject in the stagnated state.

As described above, in the first embodiment of the present invention, the representative images of one or more frames specified from a plurality of representative images displayed in a list in the display unit are set to non-display, the representative images of the same number as the representative images set to non-display are newly extracted, and the newly extracted representative images of the same number are added to the list-display of the existing representative images, where other configurations are similar to the fourth example. Thus, the effects similar to the fourth example can be benefited, the image unnecessary for the observation of the inside of the organ of the subject can be easily deleted from the representative images displayed in a list on the display unit and the representative images having the possibility of being the images useful for the observation are newly displayed in a list on the display unit in place of the deleted representative images, whereby the representative images unnecessary for the observation can be reduced form the list-display of the representative images, the possibility of displaying the representative images useful for the observation can be enhanced, and the overlook of the characteristic site of the inside of the subject can be further suppressed.

In the first embodiment, the latent images of one or more frames represented by the representative image set to non-display are set as the non-display image, and thus the displaying number of images unnecessary for the observation occupying the list-display of the latent image in the display unit can be reduced, whereby the probability of displaying the representative images unnecessary for the observation can be reduced and the possibility of displaying the in-vivo images useful for the observation can be further enhanced so that the in-vivo image group of the subject can be observed in a short period of time.

### Fifth Example

A fifth example will now be described. In the first embodiment described above, the representative image to be set to non-display is specified from a plurality of representative images displayed in a list in the list-display area 51 of the display unit 13, but in the sixth embodiment, the time position of the image set to non-display is range designated from the time position of the image indicated by the graphic bar 55 of the display unit 13, and the image corresponding to the range designated time position on the graphic bar 55 is set to non-display.

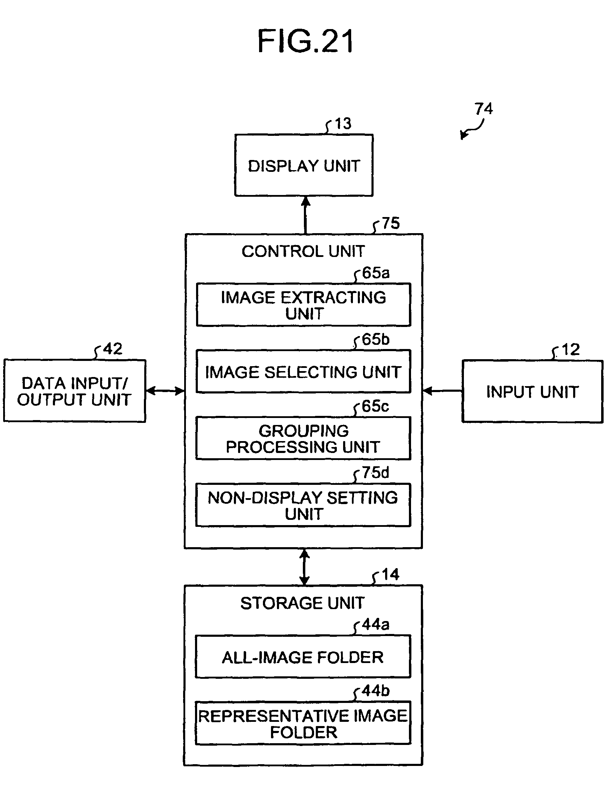

FIG. 21 is a block diagram schematically illustrating one configuration example of an image display device according to the fifth example.

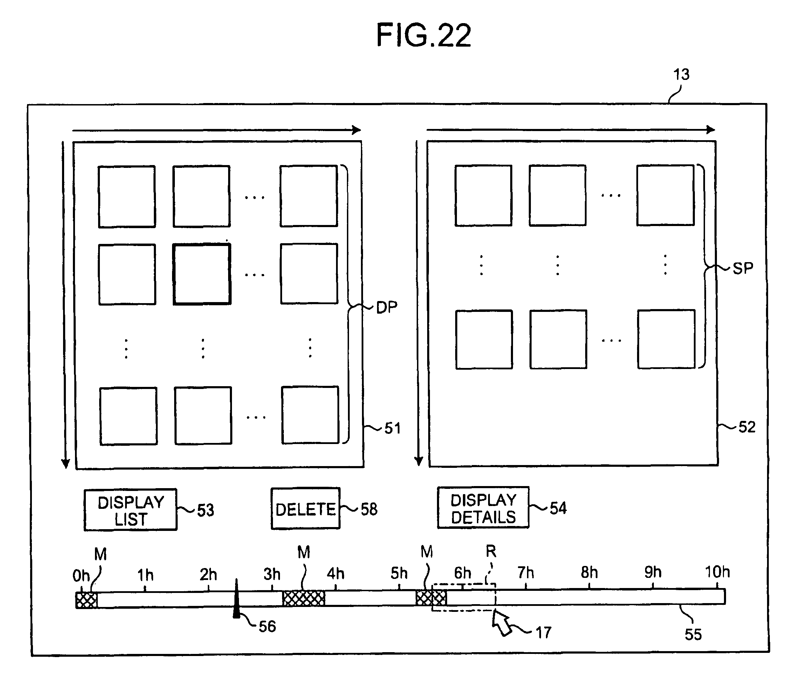

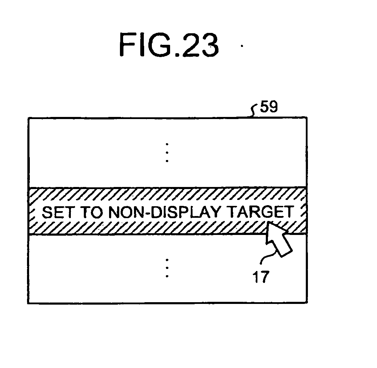

FIG. 22 is a schematic view illustrating one specific example of a display mode of the display unit according to the fifth example. FIG. 23 is a schematic view illustrating one specific example of a setup menu for setting the image to non-display. As illustrated in FIG. 21, an image display device 74 according to the fifth example includes a control unit 75 in place of the control unit 65 of the image display device 64 according to the fifth embodiment described above. In the image display device 74, the display unit 13 displays a designating range R of the image on the graphic bar 55 as illustrated in FIG. 22 and displays a setup menu 59 for setting the image to non-display, as necessary, as illustrated in FIG. 23 in response to the operation of the input unit 12. An in-vivo information acquiring system according to the sixth embodiment includes the image display device 44 in place of the image display device 4 of the in-vivo information acquiring system (see FIG. 1) according to the first embodiment described above. Other configurations are the same as any one of the first to the fourth examples and the first embodiment, and the same reference numerals are denoted for the same components.

The control unit 75 includes a non-display setting unit 75d in place of the non-display setting unit 65d of the control unit 65 in the fifth embodiment described above. The non-display setting unit 75d has a function similar to the non-display setting unit 65d in the first embodiment described above, and also has a function of setting the image within the designated range to a non-display image in accordance with the range specifying information and the non-display instructing information input by the input unit 12.

Specifically, the control unit 75 is input with the range designating information and the non-display instructing information indicating the range of the time position specified from the time position of the image indicated by the graphic bar 55 of the display unit 13 by the input unit 12. The non-display setting unit 75d adds the non-display flag F to all images corresponding to the range of the time position specified by such range designating information of the image group such as the representative images and the latent images saved in the storage unit 14, to thereby set all images within such designated range to the non-display images. The control unit 75 controls the display unit 13 so as to display the above-described mark M at the position on the graphic bar 55 corresponding to the time position of the image set to non-display by such non-display setting unit 75d, and controls the display unit 13 so as not to display such images set to non-display. The control unit 75 has a function similar to the control unit 65 of the image display device 64 according to the first embodiment described above other than the function related to the non-display setting unit 75d.

In the image display device 74 according to the fifth example, the display unit 13 displays the designating range R of the image on the graphic bar 55 as illustrated in FIG. 22 under the control of the control unit 75. The designating range R illustrates the range of the time position specified from the time positions of all images indicated by the graphic bar 55, and is displayed on the display unit 13 in response to the operation of the input unit 12 such as drag operation of the mouse. The input unit 12 inputs the range designating information indicating the range of the time position on the graphic bar 55 within the designating range R to the control unit 75 by the input operation of forming such designating range R on the graphic bar 55.

The setup menu 59 illustrated in FIG. 23 is displayed on the display unit 13 in response to the operation of the input unit 12 such as right click operation of the mouse. When the non-display setting item of the image of "set to non-display target" is selected from such setup menu 59 by click operation and the like, the input unit 12 inputs the non-display instructing information of instructing to set all images in the range of the time position corresponding to the above-described range designating information to non-display to the control unit 75.

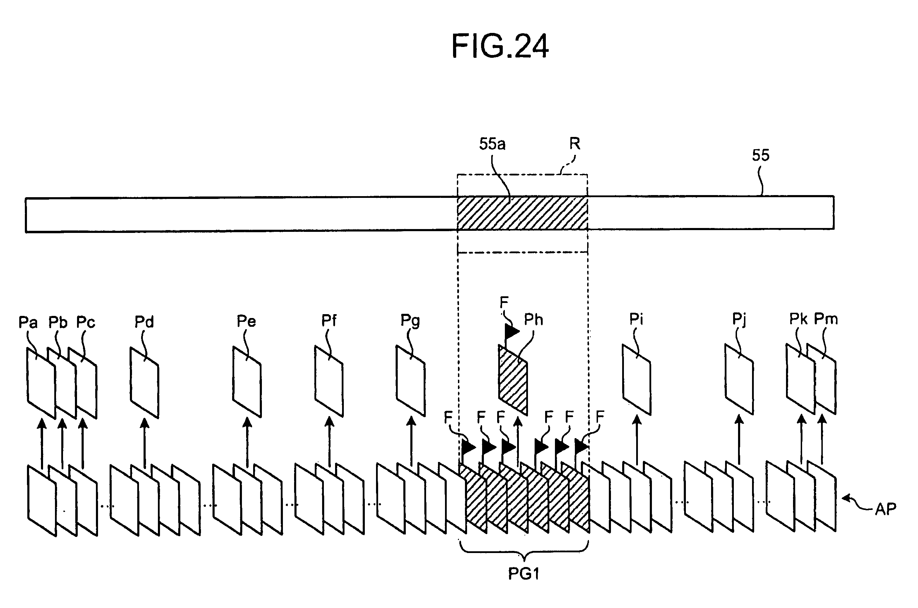

The operation of the image display device 74 according to the fifth example will be specifically described using a case of setting part of the successive image groups contained in the image group AP captured in time-series to non-display by way of example. FIG. 24 is a schematic view specifically describing the non-display setting operation of the image by the image display device according to the sixth embodiment of the present invention. The operation of the image display device 74 according to the fifth example is similar to the image display device 64 according to the first embodiment described above other than the operation of setting all images in the designated range of the time position specified on the graphic bar 55 to non-display. The operation of the image display device 74 in setting the images in the designated range of the time position specified on the graphic bar 55 to non-display will be specifically described with reference to FIGS. 22 and 24.

When the designating range R is formed on the graphic bar 55, the control unit 75 of the image display device 74 according to the fifth example is input with the range designating information indicating a time position range 55a on the graphic bar 55 within the designating range R by the input unit 12. When the non-display instructing information is input by the input unit 12 in such state, the control unit 75 excludes the successive image group PG1 corresponding to the time position range 55a indicated by the range designating information of the image group AP in the all-image folder 44a of the storage unit 14 from the non-display target of the display unit.

Specifically, as illustrated in FIG. 24, the non-display setting unit 75d adds the non-display flag F to each image in the successive image group PG1 corresponding to such time position range 55a of the image group AP to thereby set all images in the successive image group PG1 to non-display images. The control unit 75 controls the display unit 13 so as not to display the successive image group PG1 added with the non-display flag F.

If the representative image Ph is included in the non-display set successive image group PG1 as illustrated in FIG. 24, the image extracting unit 65a newly extracts the latent image having similarity of the order following the already extracted representative images Pa, Pb, Pc, Pd, Pe, Pf, Pg, Pi, Pj, Pk, Pm as the representative image in place of the non-display representative image Ph from the images not added with the non-display flag F of the image group AP. Such new representative image is displayed in a list in the list-display area 51 of the display unit 13 in addition to the existing representative images Pa, Pb, Pc, Pd, Pe, Pf, Pg, Pi, Pj, Pk, Pm, similarly to the first embodiment described above.

As described above, in the fifth example, the time position range of the specified image is designated using the graphic bar indicating the time position of each image in the image group captured in time-series, all images within the time position range are set to non-display images based on the non-display instructing information, and others are configured similar to the first embodiment. Thus, the effects similar to the first embodiment described above are benefited, the range of the time position of the image to be set to non-display can be easily designated while visually checking from the entire range of the time position of each image indicated by the graphic bar, and the successive image group corresponding to the designated range of the time position can be easily set to non-display. As a result, from the image group in time-series order, the successive image group unnecessary, as illustrated with the successive image group in which a plurality of images of the same subject is successive, for the observation of the inside of the organ of the subject can be easily excluded from the display target, so that the in-vivo image group of the subject can be observed in a shorter period of time.

### Sixth Example

A sixth example will now be described. In the fifth example described above, all images within the time position range designated from the time position of each image indicated by the graphic bar 55 of the display unit 13 are set to non-display, but in the sixth example, the time position of the image to be displayed is range designated from the time position of each image indicated by the graphic bar 55, and each image corresponding to the range designated time position on the graphic bar 55 can be selectively displayed.

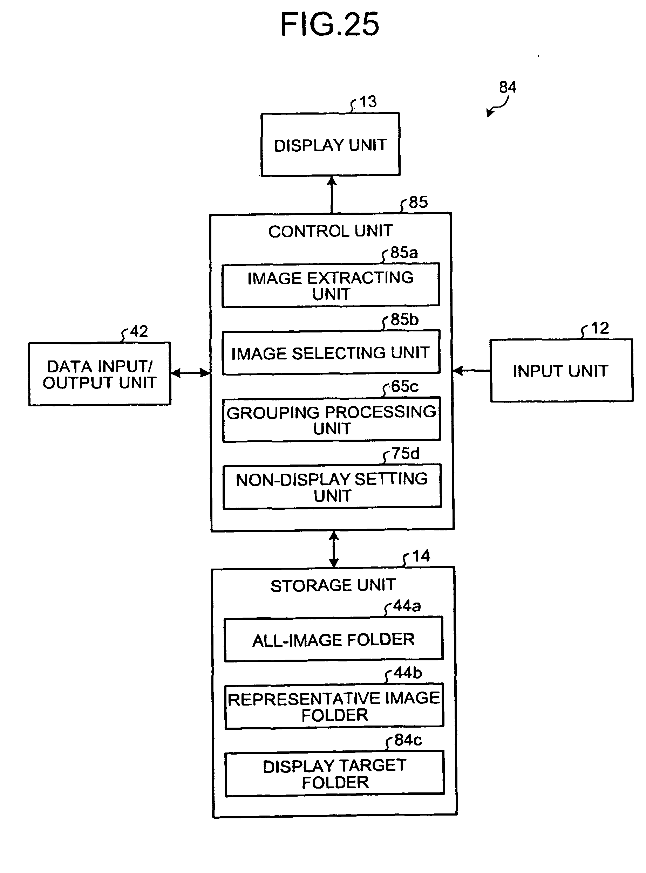

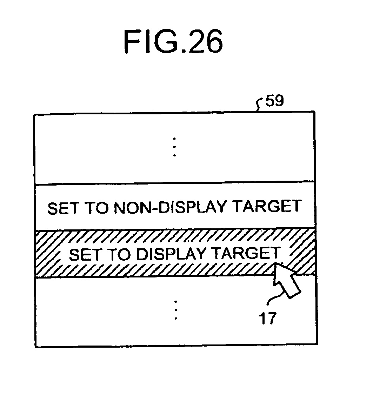

FIG. 25 is a block diagram schematically illustrating one configuration example of an image display device according to the sixth example. FIG. 26 is a schematic view illustrating one specific example of a setup menu for setting the image as a display target. As illustrated in FIG. 25, an image display device 84 according to the seventh embodiment includes a control unit 85 in place of the control unit 75 of the image display device 74 according to the sixth embodiment described above. In the image display device 84, the display unit 13 displays the setup menu 59 for setting the image as the display target or the non-display target in response to the operation of the input unit 12, as illustrated in FIG. 26. An in-vivo information acquiring system according to the seventh embodiment includes the image display device 84 in place of the image display device 4 of the in-vivo information acquiring system (see FIG. 1) according to the first example described above. Other configurations are the same as any one of the first to the fifth examples and the first embodiment, and the same reference numerals are denoted for the same components.

The control unit 85 includes an image extracting unit 85a in place of the image extracting unit 65a and an image selecting unit 85b in place of the image selecting unit 65b of the control unit 75 in the fifth example described above. Such control unit 85 has a function similar to the control unit 75 of the image display device 74 according to the sixth embodiment described above other than the function related to the image extracting unit 85a and the function related to the image selecting unit 85b.

The image extracting unit 85a has an image extracting function similar to the image extracting unit 65a described above and also has a function of extracting a representative image in the designated range based on the range designating information and the display instructing information input by the input unit 12. Specifically, when the range designating information corresponding to the above-described designating range R illustrated in FIG. 22 and the display instructing information corresponding to the display setting item of the setup menu 59 are input to the control unit 85 by the input unit 12, the image extracting unit 85a extracts the representative image existing within the range of the time position specified by the range designating information from the image group of the all-image folder 44a of the storage unit 14. In this case, the image extracting unit 85a extracts the representative image for the set displaying number based on the above-described displaying number instructing information from each image in the range of the time position specified by such range designating information. The control unit 85 controls the storage unit 14 so as to overwrite the data of the representative image for the set displaying number extracted by such image extracting unit 85a in the representative image folder 44b, and controls the display unit 13 so as to display in a list the representative images for such set displaying number in the list-display area 51.

The setup menu 59 illustrated in FIG. 26 is displayed on the display unit 13 in response to the operation of the input unit 12 such as right click operation of the mouse. When the display setting item of the image of "set to display target" is selected from such setup menu 59 by click operation and the like, the input unit 12 inputs the display instructing information of instructing to set all images in the range of the time position corresponding to the above-described range designating information to the display target to the control unit 85.

The image selecting unit 85b selects the latent images of one or more frames displayed in a list in the detailed-display area 52 of the display unit 13 from each image existing in the range of the time position specified by the above-described range designating information of the image group in the all-image folder 44a of the storage unit 14. The control unit 85 controls the storage unit 14 so as to write the data of the latent image of one or more frames selected by such image selecting unit 85b in a display target folder 84c. The image selecting unit 85b has a function similar to the above-described image selecting unit 65b other than selecting the latent image from each image existing in the range of the time position specified by such range designating information.

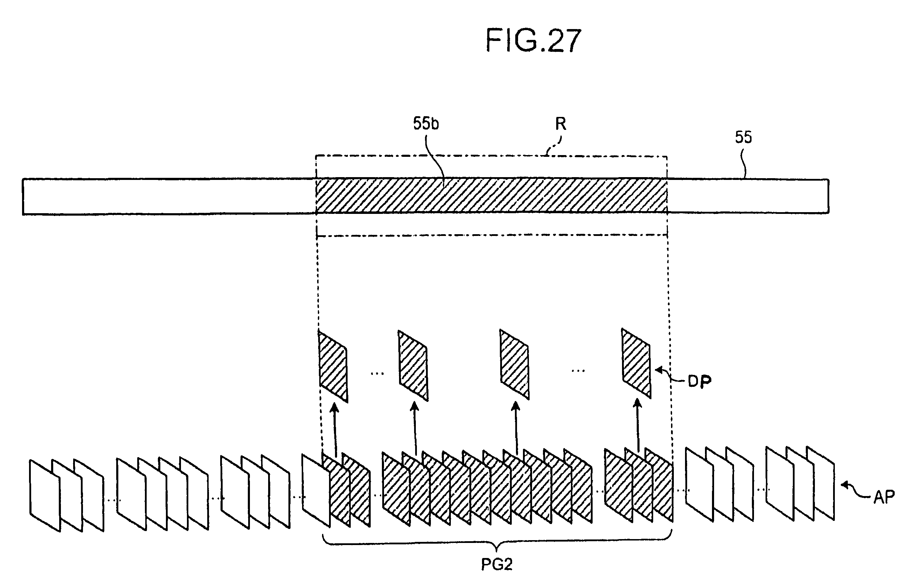

The operation of the image display device 84 according to the sixth example will be specifically described using a case of setting part of the successive image groups contained in the image group AP captured in time-series to display target by way of example. FIG. 27 is a schematic view specifically describing the operation of the image display device according to the sixth example. The operation of the image display device 84 according to the sixth example is similar to the image display device 74 according to the fifth example described above other than the operation of setting all images in the designated range of the time position on the graphic bar 55 to the display target. The operation of the image display device 84 in setting all images in the designated range of the time position specified on the graphic bar 55 to the display target will be specifically described with reference to FIGS. 25 and 27.

When the designating range R is formed on the graphic bar 55, the control unit 85 of the image display device 84 according to the sixth example is input with the range designating information indicating a time position range 55b on the graphic bar 55 within the designating range R by the input unit 12. When the display instructing information for instructing to set to the display target is input by the input unit 12 in such state, the control unit 85 sets the successive image group PG2 corresponding to the time position range 55b indicated by the range designating information of the image group AP in the all-image folder 44a of the storage unit 14 to the display target of the display unit.

Specifically, as illustrated in FIG. 27, the image extracting unit 85a extracts the representative image DP for the set displaying number described above from each image in the successive image group PG2 corresponding to the time position range 55b of the image group AP. The control unit 85 controls the storage unit 14 so as to overwrite each image data of the representative image DP extracted from such successive image group PG2 in the representative image folder 44b, and controls the display unit 13 so as to display such representative image DP in a list in the list-display area 51 described above.

When the display instructing information corresponding to the "DISPLAY DETAILS" icon 54 described above is input, the image selecting unit 85b selects the latent images of one or more frames display instructed by the display instructing information from the successive image group PG2.

As described above, in the sixth example, with all images in the time position range designated using the graphic bar indicating the time position of each image in the image group captured in time-series as the images of the display target, the representative image for the set displaying number is extracted from all images in the specified time position range, the extracted representative images for the set displaying number are displayed in a list on the display unit, and the latent images to be displayed in a list on the display unit are selected from all latent images in the time position range, and others are configured similar to the fifth example. Thus, the effects similar to the fifth example described above are benefited, the range of the time position of the image to be set to the display target can be easily specified while visually checking from the entire range of the time position of each image indicated by the graphic bar, and the successive image group corresponding to the designated range of the time position can be selectively displayed on the display unit. Therefore, the successive image group of the digestive tract sites to be observed such as the stomach, the small intestine, or the large intestine of the subject can be selectively set to the display target from the image group in time-series order, whereby the successive image group of the inside of the subject to be observed can be selectively displayed in a list and the in-vivo image group of the subject can be observed in a shorter period of time.

### Seventh example

An seventh example will now be described. In the fourth to the sixth examples and the first embodiment described above, the latent images latently existing between the representative images are displayed in a list in the detailed-display area 52 in the still image state, but in the eighth embodiment, all images to be displayed excluding the non-display set images added with the non-display flag can be moving image displayed in the detailed-display area 52.

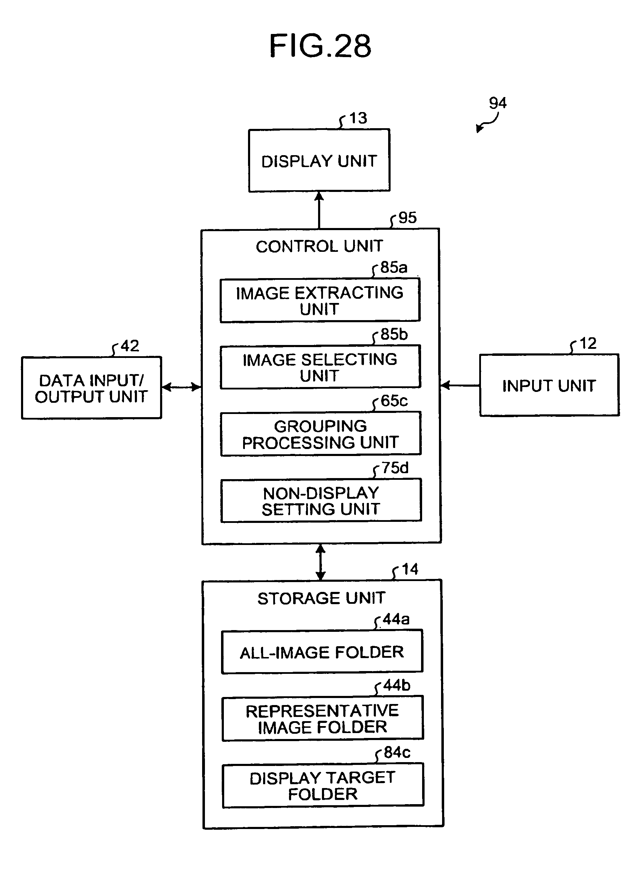

FIG. 28 is a block diagram schematically illustrating one configuration example of an image display device according to the seventh example.

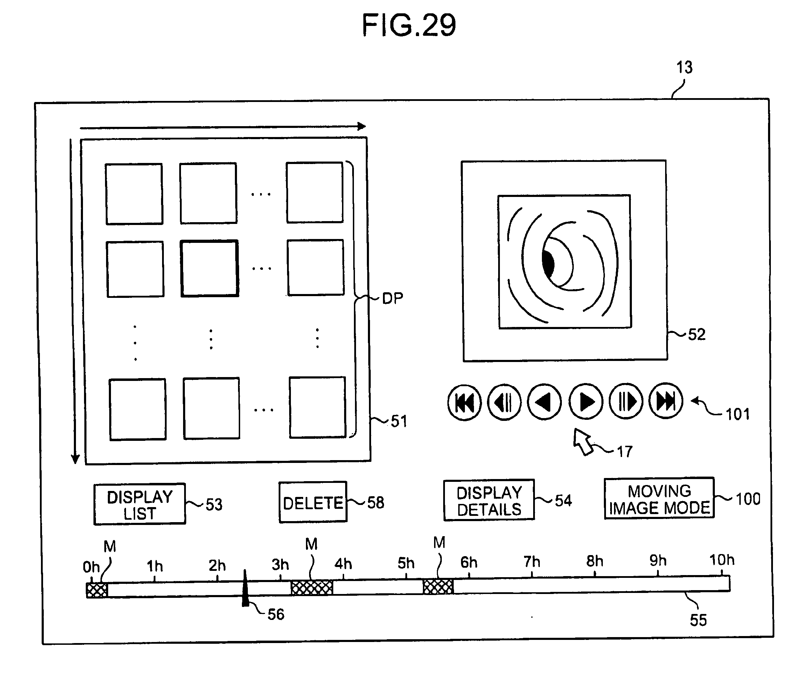

FIG. 29 is a schematic view illustrating one specific example of a display mode of the display unit according to the seventh example. As illustrated in FIG. 28, an image display device 94 according to the seventh example includes a control unit 95 in place of the control unit 85 of the image display device 84 according to the sixth example described above. In the image display device 94, the display unit 13 displays a "MOVING IMAGE MODE" icon 100, which is a GUI for instructing the setting of the moving image mode, as illustrated in FIG. 29. When the moving image mode is set, a display operation icon group 101 is displayed, which is a GUI for performing the display operation in moving image displaying the image in the detailed-display area 52. An in-vivo information acquiring system according to the seventh example includes the image display device 94 in place of the image display device 4 of the in-vivo information acquiring system (see FIG. 1) according to the first example described above. Other configurations are the same as any one of the first to the sixth examples and the first embodiment, and the same reference numerals are denoted for the same components.

When the mode setting instructing information for instructing the setting of the moving image mode is input by the input unit 12, the control unit 95 switches the display operation mode of the image from the still image mode to the moving image mode. In the moving image mode, the control unit 95 controls the display unit 13 so as to set the detailed-display area 52 described above to the display area of the moving image display, and display the display operation icon group 101 for operating the moving image display. The control unit 95 sequentially moving image reproduces each image contained in the image group of the all-image folder 44a in time-series order or the reverse order of time-series based on the moving image display instructing information corresponding to the display operation icon of such display operation icon group 101. In this case, the control unit 95 controls the display unit 13 so as to moving image display the remaining images excluding the non-display set images added with the non-display flag F described above of the image group in the all-image folder 44a in the detailed-display area 52. The image moving image displayed on the display unit 13 under the control of such control unit 95 may be only the representative image, only the latent image, or both the representative image and the latent image as long as it is an image not added with the non-display flag. The control unit 95 has a function similar to the control unit 85 of the image display device 84 according to the seventh embodiment described above other than the function related to such moving image display.

In the image display device 94 according to the seventh example, the input unit 12 inputs the mode setting instructing information of instructing the setting of the moving image mode to the control unit 95 by placing the mouse cursor 17 on the "MOVING IMAGE MODE" icon 100 and clicking the same. The input unit 12 also inputs the moving image display instructing information corresponding to the display operation icon, on which the mouse cursor 17 is placed, to the control unit 95 by placing the mouse cursor 17 on one of the display operation icons in the display operation icon group 101 and clicking the same.

As described above, in the seventh example, the remaining images other than the images set to non-display of each images in the image group captured in time-series are moving image displayed, and others are configured similar to the seventh example. Thus, the effects similar to the sixth example described above are benefited, and each remaining image other than the images unnecessary for the observation of the inside of the organ of the subject can be moving image displayed. Thus, the displaying number of images to be moving image displayed on the display unit can be reduced to a requisite minimum, and the possibility which the image of the characteristic site such as the lesion part or the bleeding part in the inside of the subject is moving image displayed can be enhanced, whereby the observation time of the in-vivo image group of the subject by the moving image reproduction can be reduced.

In the fourth to the seventh examples and the first embodiment described above, the time position of each image in the image group captured in time-series is indicated by the graphic bar 55 and the indicator 56, but is not limited thereto, and the capturing position of each image in such image group may be illustrated using a digestive tract schematic image imitating the digestive tract of the inside of the subject, which is a movement path of the capsule endoscope.

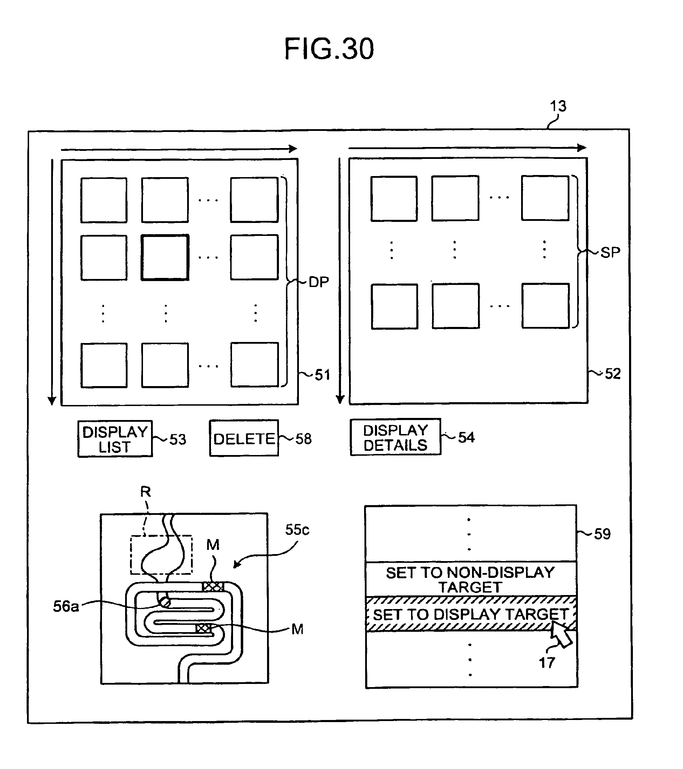

FIG. 30 is a schematic view illustrating a display mode of the display unit with the time position of each image using the digestive tract schematic image. As illustrated in FIG. 30, the display unit 13 displays a digestive tract schematic image 55c in place of the graphic bar 55 described above, and displays an indicator 56a in the inside of the digestive tract illustrated by the digestive tract schematic image 55c. The digestive tract schematic image 55c schematically illustrates the digestive tract, which is the movement path of the capsule endoscope sequentially capturing the in-vivo images of the subject in time-series. The indicator 56a moves along the digestive tract illustrated by such digestive tract schematic image 55c, and thereby illustrates the capturing position of each image in the inside of the subject. In this case, each image captured at the digestive tract position in the designating range R can be set to non-display target or display target by forming the designating range R at the desired digestive tract position in such digestive tract schematic image 55c. For instance, as illustrated in FIG. 30, if the designating range R is formed so as to surround the stomach in the digestive tract schematic image 55c, each image capturing the inside of the stomach can be excluded from the display target by selecting the non-display setting item of "set to non-display target" of the setup menu 59, and each image capturing the inside of the stomach can be selectively set to the display target by selecting the display setting item of "set to display target" of the setup menu 59. The mark M described above may be displayed at the capturing position corresponding to the non-display set image of such digestive tract schematic image 55c.

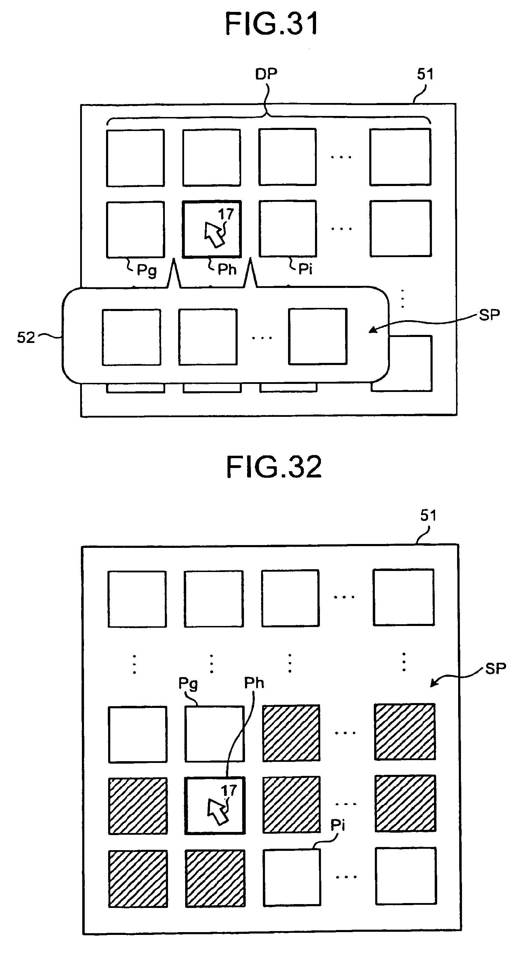

In the fourth to the seventh examples and the first embodiment described above, a plurality of representative images are displayed in a list in the list-display area 51 of the display unit 13, and the latent images of one or more frames latently existing between the representative images are displayed in a list in the detailed-display area 52, which is a display area different from the list-display area 51, but the present invention is not limited thereto. FIG. 31 is a schematic view illustrating one modified example of the detailed-display area displaying the latent images latently existing between the representative images in a list. FIG. 32 is a schematic view illustrating another mode of the list display example of the latent image.

As illustrated in FIG. 31, the image display device may display in a list the latent images of one or more frames latently existing between the representative images in a plurality of representative images displayed in a list in the list-display area 51 in the detailed-display area 52 of balloon form in a mode of pointing to the position between the representative images where the latent images of one or more frames latently exist. For instance, in FIG. 31, the detailed-display area 52 of balloon form displaying the latent image group SP in a list points to positions between the representative images Pg, Ph and the representative images Ph, Pi where the latent image group SP is latently existed. The image display device according to the present invention may display in a list the latent images in the list-display area 51 displaying the representative images in a list, as illustrated in FIG. 32. In this case, the image display device displays in a list each latent image latently existing between the representative images Pg, Ph of the latent image group SP at between the representative images Pg, Ph in the list-display area 51, and displays in a list each latent image latently existing between the representative images Ph, Pi of the latent image group SP at between the representative images Ph, Pi.

Although not illustrated, the image display device may display in a list the latent images represented by the representative image while being arrayed at the upper stage or the lower stage of such representative image, or may display in a list the latent images represented by the representative image while being arrayed at the right side or the left side of such representative image.

In the fourth to the seventh examples and the first embodiment described above, one of the plurality of representative images displayed in a list is designated when displaying the latent images latently existing between the representative images in a list, but this is not the sole case, and a plurality of representative images may be designated and the latent images represented by each specified plurality of representative images may be displayed in a list, or two representative images of the plurality of representative images displayed in a list may be designated and the latent images latently existing between the two designated representative images may be displayed in a list. In this case, such two representative images to be designated may be representative images adjacent in time-series, or may be representative images having a representative images of one or more frames in between.

In the first embodiment described above, the representative image designated from a plurality of representative images displayed in a list and each latent image represented by such representative image are set to the non-display target, but this is not the sole case, and only the designated representative image may be set to the non-display target.

Furthermore, in the fifth and sixth examples described above, all images corresponding to the designating range R formed on the graphic bar 55 are selectively set to the display target or the non-display target, but this is not the sole case, and only the representative images of all images designated by such designating range R may be selectively set to the display target or the non-display target, or only the latent image may be selectively set to the display target or the non-display target.

In the fourth to the seventh examples and the first embodiment described above, all images latently existing between the specified representative images are displayed in a list, but not limited thereto, the latent images of one or more frames of all latent images latently existing between the designated representative images may be displayed in a list, for example, the latent images may be displayed in a list in a mode of skipping by one frame, or the latent images may be displayed in a list in a mode of skipping by plural frames.

In the fourth to the seventh examples and the first embodiment described above, the representative image is extracted based on the similarity of the image between adjacent images in the image group captured in time-series, but not limited thereto, the representative image may be extracted based on the condition other than the similarity of the image between the adjacent images. For instance, the representative image may be extracted based on the frame number of each image in such image group, the representative image may be extracted based on the time data such as elapsed time from the captured time or the start of capturing of each image, the representative image may be extracted based on the capturing position of each image, the representative image may be extracted based on the movement distance of the in-vivo imaging device such as the capsule endoscope, or the representative image may be extracted based on the magnitude of the movement of the image.

In the fourth to the seventh examples and the first embodiment described above, the image group of time-series order captured by the capsule endoscope inserted into the body is illustrated, but the in-vivo imaging device for capturing such image group is not limited to the capsule endoscope, and may be other in-vivo imaging device, and for example, the image group displayed on the image display device according to the present invention may be an image group captured by the endoscope incorporating an imaging unit, etc., at a distal end of an inserting unit to be inserted inside the digestive tract of the subject.

Furthermore, in the fourth to the seventh examples and the first embodiment described above, the already displayed image group can be saved in the recording medium attached to the data input/output unit 42, but not limited thereto, the image set to non-display target of the already displayed image group may not be output to the recording medium in the data input/output unit 42, and the remaining images excluding the image of non-display target may be recorded in the recording medium of the data input/output unit 42.

In the fourth to the seventh examples and the first embodiment described above, when displaying each image in a list, each image is arrayed in time-series order from left to right on the display screen and each image is arrayed in time-series order from top to bottom, but not limited thereto, each image displayed in a list may be in a desired array mode if arrayed in time-series order, for example, each image may be arrayed in time-series order from right to left on the display screen or each image may be arrayed in time-series order from bottom to top facing the display screen. In all array modes, each array mode of the list-display area 51 of the representative image and the detailed-display area 52 of the latent image are desirably similar to each other.

According to any one of the fourth to the seventh examples and the first embodiment, an image display device capable of suppressing the overlook of the image of the characteristic site in the inside of the subject and enabling the observation of the in-vivo image group of the subject in a short period of time can be realized.

The operation of each unit in each embodiment and example described above can be realized by having a control unit configured by a CPU, etc., read out an image processing program from a computer readable recording medium, which is recorded with the image processing program according to each embodiment, and execute the program.

Additional advantages and modifications will readily occur to those skilled in the art.

## Claims

1. An image display device comprising:
an image extracting unit (15b, 15c; 45a; 65a; 85a) that extracts one or more representative images satisfying a predetermined condition from a series of images (AP) captured in time-series; and
a display unit (13, 15a/25a/35a/45/65/75/85/95) that displays the one or more representative images (P1 to P15; DP) extracted by the image extracting unit (15b, 15c; 45a; 65a; 85a) in a list, and displays information (M1 to M3; A1 to A15; SP) of an image not extracted by the image extracting unit (15b, 15c; 45a; 65a; 85a), wherein
the series of images (AP) includes one or more image groups (PeG; PG1; PG2) divided by the representative images (DP) extracted by the image extracting unit (45a; 65a; 85a), and
the display unit (13, 45/65/75/85/95) list-displays the one or more representative images (DP) extracted by the image extracting unit (45a; 65a; 85a), selects one or more latent images from the one or more image groups (PeG; PG1; PG2) divided by the representative images (DP), and list-displays the selected one or more latent images as the information (SP) of the image not extracted, wherein
the image display device further comprises
an input unit (12, 17) that inputs representative image specifying information for specifying one or more of the representative images from the list-displayed representative images (DP), wherein
the display unit (13, 45/65/75/85/95) selects one or more of the latent images from the one or more image groups (PeG; PG1; PG2) divided by the one or more representative images specified by the representative image specifying information and list-displays the selected one or more latent images as the information (SP) of the image not extracted,
the input unit (12, 17) inputs non-display instructing information for hiding an image, and
the display unit (13, 45/65/75/85/95) hides the one or more representative images specified by the representative image specifying information when the non-display instructing information is input by the input unit (12, 17),
**characterized in that**
when the non-display instructing information is input by the input unit (12, 17), the display unit (13, 45/65/75/85/95) groups one of the representative images specified by the representative image specifying information and the one or more latent images latently existing between the representative image and the adjacent representative image, which is on an earlier side in time-series, and/or between the representative image and the adjacent representative image, which is on a later side in time-series, and hides the grouped representative image and the one or more latent images.

2. The image display device according to claim 1, wherein
when the non-display instructing information is input by the input unit (12, 17), the display unit (13, 45/65/75/85/95) sets the one or more representative images specified by the representative image specifying information and the one or more latent images selected from the one or more image groups represented by the one or more representative images to non-display.

3. The image display device according to claim 1, wherein
the display unit (13, 45/65/75/85/95) extracts the representative images of the same number as that of the non-display set representative images from the latent images in the image group, and contains and displays the thus extracted representative images in the list of the representative images.

4. The image display device according to claim 1, wherein
the display unit (13, 45/65/75/85/95) list-displays the one or more latent images in the same array mode as the representative images.

## Patentansprüche

1. Bildanzeigevorrichtung mit:
einer Bildextraktionseinheit (15b, 15c; 45a; 65a; 85a), die aus einer in Zeitreihen aufgenommenen Serie von Bildern (AP) ein oder mehrere repräsentative Bilder extrahiert, die eine vorgegebene Bedingung erfüllen; und
einer Anzeigeeinheit (13, 15a/25a/35a/45/65/75/85/95), die das eine oder die mehreren repräsentativen Bilder (P1 bis P15; DP), die durch die Bildextraktionseinheit (15b, 15c; 45a; 65a; 85a) extrahiert worden sind, in einer Liste anzeigt und Informationen (M1 bis M3; A1 bis A15; SP) von einem Bild anzeigt, das durch die Bildextraktionseinheit (15b, 15c; 45a; 65a; 85a) nicht extrahiert wurde, wobei
die Serie von Bildern (AP) eine oder mehrere Bildgruppen (PeG; PG1; PG2) aufweist, die durch die repräsentativen Bilder (DP) unterteilt sind, welche durch die Bildextraktionseinheit (45a; 65a; 85a) extrahiert worden sind, und
die Anzeigeeinheit (13, 45/65/75/85/95) das eine oder die mehreren repräsentativen Bilder (DP) aufgelistet anzeigt, die durch die Bildextraktionseinheit (45a; 65a; 85a) extrahiert worden sind, eines oder mehrere latente Bilder aus der einen oder den mehreren Bildgruppen (PeG; PG1; PG2) auswählt, die durch die repräsentativen Bilder (DP) unterteilt sind, und das ausgewählte eine oder die ausgewählten mehreren latenten Bilder als die Informationen (SP) des nicht extrahierten Bildes aufgelistet anzeigt, wobei
die Bildanzeigevorrichtung des Weiteren aufweist
eine Eingabeeinheit (12, 17), die Spezifizierungsinformationen zum repräsentativen Bild eingibt, um eines oder mehrere der repräsentativen Bilder von den aufgelistet angezeigten repräsentativen Bildern (DP) zu bestimmen, wobei
die Anzeigeeinheit (13, 45/65/75/85/95) eines oder mehrere der latenten Bilder aus der einen oder den mehreren Bildgruppen (PeG; PG1; PG2) auswählt, die durch das eine oder die mehreren repräsentativen Bilder unterteilt sind, die durch die Spezifizierungsinformationen zum repräsentativen Bild bestimmt werden, und das ausgewählte eine oder die ausgewählten mehreren latenten Bilder als die Informationen (SP) des nicht extrahierten Bildes aufgelistet anzeigt,
die Eingabeeinheit (12, 17) Nichtanzeige-Anweiseinformationen zum Verbergen eines Bildes eingibt, und
die Anzeigeeinheit (13, 45/65/75/85/95) das eine oder die mehreren repräsentativen Bilder ausblendet, die durch die Spezifizierungsinformationen zum repräsentativen Bild bestimmt werden, wenn die Nichtanzeige-Anweiseinformationen durch die Eingabeeinheit (12, 17) eingegeben werden,
**dadurch gekennzeichnet, dass**
wenn die Nichtanzeige-Anweiseinformationen durch die Eingabeeinheit (12, 17) eingegeben werden, die Anzeigeeinheit (13, 45/65/75/85/95) eines der repräsentativen Bilder, das durch die Spezifizierungsinformationen zum repräsentativen Bild bestimmt wird, und das eine oder die mehreren latenten Bilder, die zwischen dem repräsentativen Bild und dem benachbarten repräsentativen Bild, das in der Zeitreihe früher erscheint, und/oder zwischen dem repräsentativen Bild und dem benachbarten repräsentativen Bild, das in der Zeitreihe später erscheint, latent vorhanden sind, gruppiert und das gruppierte repräsentative Bild und das eine oder die mehreren latenten Bilder ausblendet.

2. Bildanzeigevorrichtung nach Anspruch 1, wobei
wenn die Nichtanzeige-Anweiseinformationen durch die Eingabeeinheit (12, 17) eingegeben werden, die Anzeigeeinheit (13, 45/65/75/85/95) das eine oder die mehreren repräsentativen Bilder, die durch die Spezifizierungsinformationen zum repräsentativen Bild bestimmt werden, und das eine oder die mehreren latenten Bilder, die aus der einen oder den mehreren Bildgruppen ausgewählt werden, welche durch das eine oder die mehreren repräsentativen Bilder dargestellt werden, auf Nichtanzeigen einstellt.

3. Bildanzeigevorrichtung nach Anspruch 1, wobei
die Anzeigeeinheit (13, 45/65/75/85/95) die repräsentativen Bilder mit derselben Nummer wie die der auf Nichtanzeigen eingestellten repräsentativen Bilder aus den latenten Bildern in der Bildgruppe extrahiert und die so extrahierten repräsentativen Bilder in die Liste der repräsentativen Bilder aufnimmt und anzeigt.

4. Bildanzeigevorrichtung nach Anspruch 1, wobei
die Anzeigeeinheit (13, 45/65/75/85/95) das eine oder die mehreren latenten Bilder in demselben Array-Modus wie die repräsentativen Bilder aufgelistet anzeigt.

## Revendications

1. Dispositif d'affichage d'images comprenant :
une unité d'extraction d'images (15b, 15c ; 45a ; 65a ; 85a) qui extrait une ou plusieurs images représentatives satisfaisant une condition prédéterminée à partir d'une série d'images (AP) capturées en série chronologique ; et
une unité d'affichage (13, 15a/25a/35a/45/65/75/85/95) qui affiche la ou les plusieurs images représentatives (P1 à P15 ; DP) extraites par l'unité d'extraction d'images (15b, 15c ; 45a ; 65a ; 85a) dans une liste, et des informations d'affichage (M1 à M3 ; A1 à A15 ; SP) d'une image n'étant pas extraite par l'unité d'extraction d'images (15b, 15c ; 45a ; 65a ; 85a), dans lequel
la série d'images (AP) comprend un ou plusieurs groupes d'images (PeG ; PG1 ; PG2) divisés par les images représentatives (DP) extraites par l'unité d'extraction d'images (45a ; 65a ; 85a), et
l'unité d'affichage (13, 45/65/75/85/95) affiche sous forme de liste la ou les plusieurs images représentatives (DP) extraites par l'unité d'extraction (45a ; 65a ; 85a), sélectionne une ou plusieurs images latentes à partir du ou des plusieurs groupes d'images (PeG ; PG1 ; PG2) divisés par les images représentatives (DP), et affiche sous forme de liste la ou les plusieurs images latentes en tant qu'informations (SP) de l'image n'étant pas extraite, dans lequel
le dispositif d'affichage d'images comprend en outre
une unité d'entrée (12, 17) qui délivre en entrée des informations de spécification d'images représentatives destinées à spécifier une ou plusieurs images représentatives à partir des images représentatives affichées sous forme de liste (DP), dans lequel
l'unité d'affichage (13, 45/65/75/85/95) sélectionne une ou plusieurs images latentes à partir du ou des plusieurs groupes (PeG ; PG1 ; PG2) divisés par la ou les plusieurs images représentatives spécifiées par les informations de spécification d'images représentatives et affiche sous forme de liste la ou les plusieurs images latentes en tant qu'informations (SP) de l'image n'étant pas extraite,
l'unité d'entrée (12, 17) délivre en entrée une information d'instruction de non affichage pour cacher une image, et
l'unité d'affichage (13, 45/65/75/85/95) cache la ou les plusieurs images représentatives spécifiées par les informations de spécification d'images représentatives lorsque l'information de l'instruction de non affichage est délivrée en entrée par l'unité d'entrée (12, 17),
**caractérisé en ce que**
lorsque l'information d'instruction de non affichage est délivrée en entrée par l'unité d'entrée (12, 17), l'unité d'affichage (13, 45/65/75/85/95) groupe l'une des images représentatives spécifiées par les informations de spécification d'images représentatives et la ou les plusieurs images latentes se trouvant en latence entre l'image représentative et les images représentatives adjacentes, se trouvant au début d'une série chronologique, et/ou entre l'image représentative et l'image représentative adjacente, se trouvant à la fin d'une série chronologique, et cache l'image représentative et la ou les plusieurs images latentes groupées.

2. Dispositif d'affichage d'images selon la revendication 1, dans lequel
lorsque l'information d'instruction de non affichage est délivrée en entrée par l'unité d'entrée (12, 17), l'unité d'affichage (13, 45/65/75/85/95) fixe la ou les plusieurs images représentatives spécifiées par les informations de spécification d'images représentatives et la ou les images latentes sélectionnées à partir du ou des plusieurs groupes d'images représentés par la ou les images représentatives pour ne pas les afficher.

3. Dispositif d'affichage d'images selon la revendication 1, dans lequel
l'unité d'affichage (13, 45/65/75/85/95) extrait les images représentatives en un nombre identique à celui des images représentatives fixées à ne pas afficher à partir des images latentes dans le groupe d'images, et contient et affiche les images représentatives ainsi extraites dans la liste des images représentatives.

4. Dispositif d'affichage d'images selon la revendication 1, dans lequel
l'unité d'affichage de (13, 45/65/75/85/95) affiche sous forme de liste la ou les plusieurs images latentes dans le même mode tableau que les images représentatives.
